# EUROPÄISCHE PATENTSCHRIFT

(11) **EP 4 478 040 B1**
(45) Veröffentlichungstag und Bekanntmachung des Hinweises auf die Patenterteilung: **05.11.2025**
(21) Anmeldenummer: 24180637.1
(22) Anmeldetag: 07.06.2024
(51) Int. Cl.: G01N 27/16, G01N 33/00

(54) **VORRICHTUNG UND VERFAHREN ZUM DETEKTIEREN EINES ZIELGASES MITTELS MEHRERER DETEKTOR-KOMPENSATOR-PAARE**
APPARATUS AND METHOD FOR DETECTING A TARGET GAS USING MULTIPLE DETECTOR/CANCELLER PAIRS
DISPOSITIF ET PROCÉDÉ DE DÉTECTION D'UN GAZ CIBLE AU MOYEN DE PLUSIEURS PAIRES DE CAPTEURS-COMPENSATEURS

(30) Priorität: 15.06.2023 DE 102023115582
(43) Veröffentlichungstag der Anmeldung: 18.12.2024
(73) Patentinhaber: Drägerwerk AG & Co. KGaA, 23558 Lübeck (DE)
(72) Erfinder: Fornasiero, Livio, 23558 Lübeck (DE); Osswald, Jürgen, 23558 Lübeck (DE); Pöthig, Tom, 23558 Lübeck (DE)

(56) Entgegenhaltungen:
- WO-A1-2009/008258
- WO-A1-2009/013103
- WO-A1-2020/251931

## Beschreibung

Die Erfindung betrifft eine Gasdetektionsvorrichtung und ein Gasdetektionsverfahren, welche dazu ausgestaltet sind, in einer Gasprobe das Vorhandensein mindestens eines brennbaren Zielgases zu detektieren und / oder die Konzentration des Zielgases in der Gasprobe zu ermitteln.

Bekannt geworden sind Gasdetektionsvorrichtungen, die einen Detektor und einen Kompensator umfassen. Indem jeweils eine elektrische Spannung angelegt wird, werden der Detektor und der Kompensator erhitzt. Der erhitzte Detektor oxidiert brennbares Zielgas. Eine Detektionsgröße, die mit der Zielgas-Konzentration korreliert, wird gemessen. Der erhitzte Kompensator vermag in geringerem Umfang als der Detektor oder sogar überhaupt nicht brennbares Zielgas zu oxidieren. Weil idealerweise der Detektor und der Kompensator gleichartig auf Umgebungsbedingungen reagieren, ermöglicht es der Kompensator, den Einfluss von Umgebungsbedingungen auf die Detektionsgröße zu kompensieren. Eine solche Vorrichtung wird auch als "Wärmetönungs-Sensor" bezeichnet. Um auch bei geringeren Temperaturen ausreichend Zielgas zu oxidieren, umfasst der Detektor häufig katalytisches Material. Daher wird eine solche Vorrichtung auch als "katalytischer Sensor" bezeichnet.

WO2020/251931 offenbart eine Gasdetektionsvorrichtung mit drei beheizten Detektoren, an denen ein Zielgas katalytisch oxidiert wird. Ein Kompensator ohne Katalysator wird ebenfalls beheizt, oxidiert das Zielgas aber nicht.

Der Erfindung liegt die Aufgabe zugrunde, eine Gasdetektionsvorrichtung und ein Gasdetektionsverfahren bereitzustellen, die bei gleichem Energieverbrauch eine höhere Zuverlässigkeit und / oder bei gleicher Zuverlässigkeit einen geringeren Energieverbrauch als bekannte Gasdetektionsvorrichtungen und Gasdetektionsverfahren mit jeweils einem Detektor und einem Kompensator bewirken.

Die Aufgabe wird durch eine Gasdetektionsvorrichtung mit den Merkmalen des Anspruchs 1 und durch ein Gasdetektionsverfahren mit den Merkmalen des Anspruchs 16 gelöst. Vorteilhafte Ausgestaltungen der erfindungsgemäßen Gasdetektionsvorrichtung sind, soweit sinnvoll, auch vorteilhafte Ausgestaltungen des erfindungsgemäßen Gasdetektionsverfahrens und umgekehrt.

Die erfindungsgemäße Gasdetektionsvorrichtung vermag in einer Gasprobe über das Vorhandensein mindestens eines brennbaren Zielgases zu entscheiden. Alternativ oder zusätzlich vermag die Gasdetektionsvorrichtung die Konzentration des oder mindestens eines Zielgases in der Gasprobe zu ermitteln. Optional vermag die Gasdetektionsvorrichtung die summierten Konzentrationen von mehreren Zielgasen in der Gasprobe zu ermitteln. Das erfindungsgemäße Gasdetektionsverfahren wird unter Verwendung einer erfindungsgemäßen Gasdetektionsvorrichtung durchgeführt.

Die erfindungsgemäße Gasdetektionsvorrichtung umfasst eine Detektions-Anordnung. Die Detektions-Anordnung umfasst mindestens drei Detektionseinheiten. Der Begriff "Detektionseinheit" kann einen Detektor oder einen Kompensator bezeichnen. Mindestens zwei verschiedene Detektionseinheiten der Detektions-Anordnung sind Detektoren. Mindestens eine weitere Detektionseinheit ist ein Kompensator. Dadurch werden mindestens zwei verschiedene Detektor-Kompensator-Paare gebildet. Jedes Detektor-Kompensator-Paar umfasst jeweils genau einen Detektor und genau einen Kompensator und wird manchmal abkürzend als ein "Paar" bezeichnet.

Erfindungsgemäß ist die Anzahl der Detektoren größer als die Anzahl der Kompensatoren. Der oder mindestens ein Kompensator gehört daher zu mindestens zwei verschiedenen Detektor-Kompensator-Paaren. Möglich ist, dass ein Kompensator sogar zu mindestens drei verschiedenen Detektor-Kompensator-Paaren gehört.

Die erfindungsgemäße Gasdetektionsvorrichtung vermag jedes Paar unabhängig von jedem anderen Paar einzuschalten, d.h. zu bestromen, und wieder auszuschalten. Erfindungsgemäß ist zu jedem Zeitpunkt während eines Einsatzes höchstens ein Detektor-Kompensator-Paar eingeschaltet. Das oder jedes andere Detektor-Kompensator-Paar ist ausgeschaltet. Möglich ist, dass in einem Ruhezustand der Gasdetektionsvorrichtung jedes Paar ausgeschaltet ist. Möglich ist auch, dass in einem ersten Zeitraum ein erstes Paar eingeschaltet ist, dann in einem Zwischenzeitraum kein Paar eingeschaltet ist und anschließend ein zweites Paar eingeschaltet ist, wobei das zweite Paar vom ersten Paar verschieden ist oder das gleiche Paar wie das erste Paar ist.

Falls ein Paar eingeschaltet ist, so liegt dauerhaft oder wenigstens zeitweise eine elektrische Spannung am Detektor an. Außerdem liegt dauerhaft oder wenigstens zeitweise eine elektrische Spannung am Kompensator an. Die Bezeichnung "zeitweise" umfasst insbesondere die Ausgestaltung, dass die elektrische Spannung jeweils gepulst angelegt wird, was elektrische Energie einspart, verglichen mit einer Ausgestaltung, bei der die Spannung dauerhaft anliegt. Die Pulsfrequenz, die Pulsdauer und / oder der Tastgrad der gepulsten Spannung, die an einem Detektor anliegt, kann mit der Pulsfrequenz, der Pulsdauer und / oder dem Tastgrad der Spannung, die an einem Kompensator anliegt, übereinstimmen oder sich von dieser unterscheiden.

Bevorzugt wird ein Paar dadurch eingeschaltet, dass sowohl an den Detektor als auch an den Kompensator jeweils eine elektrische Spannung angelegt wird, d.h. das Paar bestromt wird. Die beiden elektrischen Spannungen können den gleichen Wert oder zwei unterschiedliche Werte annehmen. Die elektrische Spannung, die dauerhaft oder zeitweise an einer Detektionseinheit eines eingeschalteten Paars anliegt, bewirkt, dass die Detektionseinheit sich erhitzt.

Eine Detektionseinheit umfasst ein heizendes Segment. Falls Spannung an die Detektionseinheit angelegt wird, so fließt elektrischer Strom, und dieses heizende Segment wird erhitzt. Ein Detektor unterscheidet sich wie folgt von einem Kompensator: Eine Erhitzung des Detektors bewirkt eine Oxidierung von Zielgas - natürlich nur dann, wenn sich mindestens ein oxidierbares Zielgas mit ausreichend großer Konzentration in der Umgebung des Detektors befindet. Durch das Oxidieren wird Wärmeenergie freigesetzt, und der Detektor wird weiter erhitzt. Eine Erhitzung des Kompensators bewirkt hingegen eine geringere Oxidierung als die Erhitzung eines Detektors. In einer Ausgestaltung bewirkt die Erhitzung des Kompensators überhaupt kein Oxidieren von Zielgas.

Jede Detektoreinheit und damit jedes Detektor-Kompensator-Paar weist jeweils eine Detektionsgröße auf. Diese Detektionsgröße korreliert mit der Wärmeenergie, die dadurch freigesetzt wird, dass der Detektor brennbares Zielgas oxidiert, und daher mit der Zielgas-Konzentration. Die Detektionsgröße eines Paars hängt in einer Ausgestaltung vom jeweiligen elektrischen Widerstand oder einer sonstigen elektrischen Größe der beiden Detektionseinheiten ab. Bekanntlich hängt der elektrische Widerstand eines elektrisch leitenden Bauteils in vielen Fällen von der Temperatur des Bauteils ab. Auch die jeweilige Temperatur selber oder die jeweils zugeführte elektrische Leistung, die zum Anheizen der heizenden Segmente erforderlich ist, können eine Detektionsgröße sein. In der Regel weisen alle Detektor-Kompensator-Paare die gleiche Detektionsgröße auf, wobei die Werte dieser Detektionsgröße sich für verschiedene Paare voneinander unterscheiden oder wenigstens unterscheiden können. Wenn eine Detektionseinheit eines Paars Zielgas oxidiert, so erhitzt sich dadurch die Detektionseinheit weiter, und dadurch verändert sich der Wert der Detektionsgröße des Paars. Ein Beispiel: Bekanntlich steigt der elektrische Widerstand eines elektrisch leitenden Bauteils mit der Temperatur des Bauteils. Ein anderes Beispiel: Weil beim Oxidieren von brennbarem Zielgas Wärmeenergie freigesetzt wird, wird umso weniger elektrische Leistung benötigt, um einen Detektor auf eine bestimmte Temperatur zu bringen, je größer die Zielgas-Konzentration vor dem Oxidieren ist.

Die Detektionsgröße eines Detektor-Kompensator-Paars wird von der jeweiligen Temperatur der beiden Detektionseinheiten beeinflusst. Das Oxidieren des Zielgases setzt Wärmeenergie frei. Die freigesetzte Wärmeenergie und daher die mit der freigesetzten Wärmeenergie korrelierende Detektionsgröße sind ein Maß für die Zielgas-Konzentration.

Die erfindungsgemäße Gasdetektionsvorrichtung umfasst weiterhin eine Messeinheit. Diese Messeinheit vermag für jedes eingeschaltete Detektor-Kompensator-Paar jeweils zu messen, welchen Wert die Detektionsgröße aktuell für dieses Paar annimmt. Beispielsweise misst die Messeinheit die jeweilige Spannung am Detektor und am Kompensator oder auch eine Brückenspannung einer Wheatstone'schen Messbrücke. Bevorzugt erzeugt die Messeinheit für jedes Paar jeweils einen zeitlichen Verlauf der Detektionsgröße.

Die erfindungsgemäße Gasdetektionsvorrichtung umfasst weiterhin eine signalverarbeitende Auswerteeinheit, die von der Messeinheit ein Signal empfängt und automatisch auswertet. Für jedes eingeschaltete Paar empfängt die Auswerteeinheit daher mindestens einen gemessenen Wert, den die Detektionsgröße für das Paar annimmt, beispielsweise den Wert, den der Detektor des Paares annimmt, sowie einen gemessenen Wert, den die Detektionsgröße für den Kompensator des Paars annimmt, oder den Wert, den eine optionale Brückenschaltung des Paars annimmt. Unter Verwendung des oder mindestens eines Werts für das Paar, z.B. der beiden Werte für den Detektor und für den Kompensator, entscheidet die Auswerteeinheit automatisch, ob ein Zielgas mit einer Konzentration oberhalb einer vorgegebenen unteren Detektionsschranke vorliegt, und / oder leitet einen Schätzwert für die Zielgas-Konzentration her.

Falls beispielsweise ein Maß für den elektrischen Widerstand als Detektionsgröße gemessen wird, so ist der Schätzwert für die Zielgas-Konzentration in vielen Fällen umso größer, je größer der elektrische Widerstand des Detektors ist, und manchmal umso größer, je kleiner der elektrische Widerstand des Kompensators ist. Insbesondere ist der Schätzwert umso größer, je größer die Differenz zwischen den beiden elektrischen Widerständen ist.

Wie bereits erwähnt, lässt sich jedes Detektor-Kompensator-Paar unabhängig von jedem anderen Detektor-Kompensator-Paar einschalten und wieder ausschalten. Bei eingeschaltetem Paar liegt sowohl am Detektor als auch am Kompensator des Paars jeweils eine elektrische Spannung an, und zwar bevorzugt gepulst. Bei ausgeschaltetem Paar liegt weder am Detektor noch am Kompensator eine elektrische Spannung an, auch nicht gepulst.

Die Gasdetektionsvorrichtung vermag für jedes Paar die folgenden Schritte durchzuführen:
- das Paar einzuschalten,
- für das eingeschaltete Paar zu bewirken, dass die Detektionsgröße des Paars gemessen wird und abhängig von der gemessenen Detektionsgröße über das Vorhandensein und / oder die Konzentration von Zielgas zu entscheiden, und
- das Paar wieder auszuschalten.

Das erfindungsgemäße Merkmal, dass höchstens ein Paar eingeschaltet ist, spart elektrische Energie ein verglichen mit einer Ausgestaltung, bei der wenigstens zeitweise zwei Paare gleichzeitig eingeschaltet sind.

Auch Umgebungsbedingungen beeinflussen in der Regel die Temperatur des eingeschalteten Detektors, insbesondere die Temperatur, die Feuchte und der Luftdruck in der Umgebung. Diese Umgebungsbedingungen wirken idealerweise in gleicher Weise auf den Detektor wie auf den Kompensator. Der Einfluss der Umgebungsbedingungen lässt sich dank des Kompensators mit Hilfe des Werts, den die Detektionsgröße für den Kompensator oder für das Paar annimmt, durch die Konstruktion und / oder rechnerisch bis zu einem gewissen Grad kompensieren.

Auf der Oberfläche eines Detektors können sich Substanzen absetzen, insbesondere Siloxane und H₂S. Der Detektor wird "vergiftet". Dadurch vermag der Detektor weniger brennbares Zielgas zu oxidieren, und bei gleicher Zielgas-Konzentration setzt der Detektor mit zunehmender Vergiftung immer weniger Wärmeenergie frei, im Extremfall überhaupt keine Wärmeenergie. Weil erfindungsgemäß mindestens zwei Detektoren vorhanden sind, lässt sich die Lebensdauer der Gasdetektionsvorrichtung verlängern, verglichen mit einer Ausgestaltung mit nur einem Detektor.

Möglich, aber dank der Erfindung nicht erforderlich ist es, ein Filter zwischen der Detektions-Anordnung und einem zu überwachenden räumlichen Bereich zu positionieren, wobei dieses Filter den Zufluss von Schadgasen reduziert. Ein solches Filter reduziert oft die Verwendungsmöglichkeiten einer Gasdetektionsvorrichtung, weil das Filter den Zufluss von manchen Zielgasen reduzieren oder sogar unterbinden kann und die Gasdetektionsvorrichtung daher diese Zielgase nicht zu detektieren vermag. Außerdem kann ein Filter sich zusetzen und eine Gasprobe langsamer oder sogar überhaupt nicht durchlassen.

In der Regel wird der oder jeder Kompensator weniger stark vergiftet als jeder Detektor. Der wesentliche Grund ist, dass der Kompensator weniger Zielgas als der Detektor oxidiert und daher weniger stark durch Ablagerungen vergiftet wird. Insbesondere deshalb ist das erfindungsgemäße Merkmal von Vorteil, dass derselbe Kompensator der erfindungsgemäßen Gasdetektionsvorrichtung Bestandteil von mindestens zwei verschiedenen Paaren ist. Die erfindungsgemäße Gasdetektionsvorrichtung erfordert weniger Detektionseinheiten und oft auch weniger elektrische Energie als eine Gasdetektionsvorrichtung, die mehrere Detektoren und genauso viele Kompensatoren wie Detektoren aufweist.

In manchen Fällen lässt sich Zielgas mit einer ausreichend hohen Konzentration auch dann noch entdecken, wenn jeder Detektor stark vergiftet ist. In einer Ausgestaltung wird nämlich der Wert gemessen, den die Detektionsgröße für den Kompensator annimmt. Viele Zielgase haben eine größere Wärmeleitfähigkeit als Luft, so dass der Kompensator durch ein Zielgas abgekühlt wird, was die Detektionsgröße des Kompensators und damit die oder eine Detektionsgröße des Paars verändert, selbst wenn der Detektor vollständig vergiftet ist. Der Einfluss durch die verringerte Wärmeleitfähigkeit ist aber viel geringer als der Einfluss der freigesetzten Wärmeenergie auf den Detektor durch das Oxidieren von Zielgas.

Erfindungsgemäß gibt es mehr Detektoren als Kompensatoren. Dieses Merkmal hat insbesondere folgende Vorteile:
- Wie bereits dargelegt, vergiftet in der Regel jeder Detektor schneller als der oder jeder Kompensator, und zwar insbesondere, weil sich als Folge des Oxidierens Ablagerungen auf dem Detektor bilden. Daher führt diese Ausgestaltung in vielen Fällen zu einer längeren Lebensdauer und / oder zu einem geringeren Energieverbrauch als eine Gasdetektionsvorrichtung, die genauso viele Kompensatoren wie Detektoren aufweist.
- Möglich ist, dass die Detektionseinheiten flache Bauteile sind, die insbesondere auf eine Platine aufgebracht sind. In vielen Fällen sind die Herstellkosten umso größer, je mehr Fläche die Bauteile auf dieser Platine einnehmen. Das erfindungsgemäße Merkmal, dass weniger Kompensatoren als Detektoren vorhanden sind, spart Platz und daher oft Herstellkosten ein verglichen mit einer Gasdetektionsvorrichtung, die genauso viele Kompensatoren wie Detektoren und genauso viele Detektoren wie eine erfindungsgemäße Gasdetektionsvorrichtung umfasst.

Ausgestaltungen der Erfindungen legen verschiedene Möglichkeiten fest, in welcher Reihenfolge und mit welcher Häufigkeit Paare ein- und ausgeschaltet werden..
In einer Ausgestaltung werden in einem Einsatz-Zeitraum die Detektor-Kompensator-Paare so eingeschaltet und ausgeschaltet, dass insgesamt, also gemessen über den gesamten Einsatz-Zeitraum hinweg, alle Paare gleich lang eingeschaltet und gleich lang ausgeschaltet sind. In einer anderen Ausgestaltung ist ein erstes Detektor-Kompensator-Paar insgesamt für eine längere Zeitdauer eingeschaltet als ein zweites Detektor-Kompensator-Paar. In einer Fortbildung oder Abwandlung ist im Einsatz-Zeitraum das erste Paar N1 mal eingeschaltet, das zweite Paar N2 mal. N1 und N2 sind zwei Anzahlen, N2 ist mindestens 1, bevorzugt mindestens 2, und N1 ist größer als N2. Bevorzugt verstreicht zwischen dem Zeitpunkt, an dem ein Paar eingeschaltet wird, und dem Zeitpunkt, an dem dieses Paar wieder ausgeschaltet wird, jedes Mal die gleiche Zeitspanne. Das Paar ist also stets für die gleiche Aktivierungs-Zeitspanne eingeschaltet. Möglich sind aber auch unterschiedliche Aktivierungs-Zeitspannen für ein Paar oder auch für zwei verschiedene Paare.

In einer Realisierungsform führt die Gasdetektionsvorrichtung im Einsatz-Zeitraum mindestens einmal eine Abfolge umfassend die folgenden Schritte durch: Zunächst wird das erste Detektor-Kompensator-Paar N-mal eingeschaltet und wieder ausgeschaltet, während das zweite Detektor-Kompensator-Paar durchgehend ausgeschaltet bleibt. Dann wird das zweite Paar einmal eingeschaltet und wieder ausgeschaltet. Hierbei ist N eine Anzahl größer als 1. Das erste Paar wird also in der Regel stärker belastet als das zweite Paar.

Diese Ausgestaltungen führen in vielen Fällen dazu, dass der Detektor des zweiten Paars langsamer vergiftet wird als der Detektor des ersten Paars. Falls der Detektor des ersten Paars so stark vergiftet ist, dass er nicht mehr ausreichend zuverlässig oxidiert, so lässt sich in vielen Fällen ein Zielgas noch mit dem zweiten Paar detektieren. Wie bereits dargelegt, kann derselbe Kompensator sowohl zum ersten Paar als auch zum zweiten Paar gehören.

Weiter oben wurden mehrere Ausgestaltung beschrieben, bei denen das erste Paar länger eingeschaltet ist als das zweite Paar. Das erste Paar ist daher auch stärker belastet als das zweite Paar, und zumindest sein Detektor wird in vielen Fällen stärker vergiftet als der Detektor des zweiten Paars. Die beiden Paare liefern bevorzugt jeweils einen Zielgas-Konzentrations-Schätzwert. In der Regel ändert sich eine Zielgas-Konzentration zwischen eine Aktivierungs-Zeitspanne des ersten Paars und der nachfolgenden Aktivierungs-Zeitspanne des zweiten Paars nicht signifikant.

In einer Ausgestaltung vermag die Auswerteeinheit diese beiden Zielgas-Konzentrations-Schätzwerte von zwei Paaren miteinander zu vergleichen. Wenn diese beiden Schätzwerte sich um mehr als eine Schranke voneinander unterscheiden, so ist dies ein Indiz dafür, dass ein paar defekt ist. Als Reaktion löst die Auswerteeinheit - optional nach einer Überprüfung - einen der folgenden beiden Schritte aus:
- Weiterhin werden die jeweiligen Detektionsgrößen-Werte der beiden Paare verwendet. Der Wert eines Parameter wird aber automatisch verändert. Die Auswerteeinheit verwendet diesen Parameter-Wert, um abhängig von dem gemessenen Detektionsgrößen-Wert des ersten Paars über das Vorhandensein von Zielgas zu entscheiden und / oder um die Zielgas-Konzentration zu messen und einen Zielgas-Konzentrations-Schätzwert herzuleiten.
- Das erste Paar wird abgeschaltet und bis zu einer Überprüfung oder einem Austausch nicht mehr eingeschaltet.

Diese Ausgestaltung ermöglicht es, dass die Gasdetektionsvorrichtung sich selbst mit relativ hoher Zuverlässigkeit automatisch überprüft. Falls die beiden Zielgas-Konzentrations-Schätzwerte der beiden Paare um mehr als die Schranke voneinander abweichen, so ist dies ein Indiz dafür, dass ein Detektor relativ stark vergiftet ist. Mit einer Plausibilitätsprüfung lässt sich in vielen Fällen automatisch feststellen, ob die Abweichung zwischen den beiden Schätzwerten durch eine Vergiftung eines Detektors oder durch einen sonstigen Fehler verursacht ist, z.B. durch eine unterbrochene elektrische Kontaktierung. Weil der Detektor des ersten Paars gemäß der Ausgestaltung länger eingeschaltet ist als der Detektor des zweiten Paares, ist in aller Regel der Detektor des ersten Paars der vergiftete Detektor und nicht der des zweiten Paars. Ein sonstiger Fehler kann natürlich das erste Paar oder das zweite Paar betreffen.

In einer Ausgestaltung weist mindestens ein Paar eine Detektionsgröße auf, die sowohl von der Temperatur des Detektors als auch von der Temperatur des Kompensators beeinflusst wird. Dies gilt z.B. dann, wenn eine Brückenschaltung verwendet wird. In einer anderen Ausgestaltung weist mindestens ein Paar zwei verschiedene Detektionsgrößen auf, nämlich eine Detektionsgröße des Detektors und eine Detektionsgröße des Kompensators. Diese beiden Detektionsgrößen können unabhängig voneinander zeitlich variieren. Die Messeinheit vermag beide Detektionsgrößen zu messen.

In einer Ausgestaltung liegt dann, wenn ein Paar eingeschaltet ist, durchgehend sowohl am Detektor als auch am Kompensator eine elektrische Spannung an, bevorzugt eine gepulste elektrische Spannung. In einer abweichenden Ausgestaltung liegt dann, wenn ein Paar eingeschaltet ist, nur zeitweise am Detektor eine elektrische Spannung an und nur zeitweise am Kompensator eine elektrische Spannung an. Wenn am Detektor eine elektrische Spannung anliegt, wird die Detektionsgröße des Detektors gemessen. Wenn am Kompensator eine elektrische Spannung anliegt, wird die Detektionsgröße des Kompensators gemessen. Anschließend werden die beiden gemessenen Werte der beiden Detektionsgrößen miteinander verglichen, um über das Vorhandensein eines Zielgases zu entscheiden und / oder um die Zielgas-Konzentration zu ermitteln. In manchen Fällen belastet diese Ausgestaltung den Detektor weniger und / oder spart elektrische Energie ein verglichen mit einer Ausgestaltung, bei der wenigstens zeitweise gleichzeitig sowohl am Detektor als auch im Kompensator jeweils eine elektrische Spannung anliegt.

In einer Fortbildung dieser Ausgestaltung liegt auch dann, wenn das Paar eingeschaltet ist, zu einem Zeitpunkt eine elektrische Spannung nur an einem der beiden Detektionseinheiten an, also entweder am Detektor oder am Kompensator, aber nicht an beiden Detektionseinheiten. Auch bei dieser Fortbildung kann die elektrische Spannung gepulst anliegen. Diese Ausgestaltung spart elektrische Energie ein.

Erfindungsgemäß ist ein Paar in einer Aktivierungs-Zeitspanne eingeschaltet, und in dieser Aktivierungs-Zeitspanne ist das oder jedes andere Paar ausgeschaltet. In einer Realisierungsform der gerade beschriebenen Ausgestaltung liegt in dieser Aktivierungs-Zeitspanne am Kompensator kürzer eine elektrische Spannung an als am Detektor. Besonders bevorzugt misst die Messeinheit in der Aktivierungs-Zeitspanne nur einmal die Detektionsgröße für den Kompensator, aber mindestens zweimal die Detektionsgröße für den Detektor. Diese Realisierungsform spart noch mehr elektrische Energie ein und berücksichtigt den Umstand, dass der Kompensator im Wesentlichen von Umgebungsbedingungen beeinflusst wird und diese Umgebungsbedingungen sich in der Regel nur langsam ändern, während eine Zielgas-Konzentration und damit die Detektionsgröße des Detektors sich rascher ändern können.

Bevorzugt erstreckt die Detektions-Anordnung sich in einer Ebene. Die maximale Abmessung der Detektions-Anordnung senkrecht zu dieser Ebene beträgt gemäß dieser bevorzugten Ausgestaltung höchstens 20% der maximalen Ausdehnung in der Ebene. Besonders bevorzugt beträgt die senkrechte Ausdehnung höchstens 10%, insbesondere höchstens 5%. Beispielsweise erstreckt sich eine Platine in dieser Ebene, und die Detektionseinheiten sind auf und / oder in dieser Platine angeordnet. In eine Betrachtungsrichtung senkrecht auf der Ebene sind die Detektionseinheiten überlappungsfrei angeordnet, bevorzugt in mindestens einer Reihe, optional in einem Rechteck mit mindestens zwei Reihen. Bevorzugt ist der oder jeder Kompensator zwischen jeweils zwei Detektoren angeordnet. In einer Ausgestaltung sind die Detektionseinheiten in einem Rechteck mit K Zeilen und L Spalten angeordnet, wobei K >= 3 und L >= 3 gilt und wobei der oder jeder Kompensator im Inneren des Rechtecks angeordnet ist. Möglich ist auch, dass die Detektionseinheiten auf mindestens zwei Platinen verteilt sind. Die beiden Platinen können sich in derselben Ebene erstrecken oder in zwei verschiedenen Ebenen, wobei die beiden verschiedenen Ebenen bevorzugt parallel zueinander sind. Bevorzugt fließt eine Gasprobe um die beiden Platinen herum.

Eine solche flache Bauart verringert die thermische Masse der Detektoren und Kompensatoren verglichen mit z.B. kugelförmigen Bauteilen (Pellistoren). Daher erreichen die Detektionseinheiten schneller eine jeweils gewünschte Betriebstemperatur. Dies wiederum ermöglicht es, die Paare für kürzere Zeiträume einzuschalten, beispielsweise mit kürzeren Pulsen und / oder längeren Abständen zwischen den einzelnen Pulsen und / oder kleineren Tastgraden. Dies wiederum reduziert den Verbrauch an elektrischer Energie, was insbesondere bei einem Gerät mit eigener Spannungsversorgung wünschenswert ist. Alternativ lässt sich bei gleichem Energieverbrauch eine höhere Abtastrate erzielen. Außerdem spart diese Ausgestaltung oft Bauraum und / oder Gewicht ein.

Die gerade beschriebene flache Bauart führt in vielen Fällen in Verbindung mit einer gepulsten Spannung und kürzeren Pulsen dazu, dass ein Detektor eine höhere Lebensdauer hat, weil er weniger vergiftet wird, verglichen mit einer Ausgestaltung, bei der dauerhaft eine elektrische Spannung am eingeschalteten Detektor anliegt oder die Pulse länger sein müssen, um ein beispielsweise kugelförmiges Bauteil ausreichend zu erhitzen. Oft lässt die Detektions-Anordnung sich außerdem leichter produzieren als eine Detektions-Anordnung mit z.B. kugelförmigen Detektionseinheiten. Dies gilt insbesondere bei einer stark automatisierten Produktion.

In einer Ausgestaltung sind alle Detektionseinheiten in derselben Ebene angeordnet. Weil die Detektionseinheiten überlappungsfrei in dieser Ebene angeordnet sind, erreicht eine Gasprobe, die auf das oder ein Zielgas zu untersuchen ist, relativ rasch alle Detektionseinheiten. Falls zwei Detektionseinheiten sich überlappen würden, so würde in vielen Fällen mehr Zeit verstreichen, bis das Zielgas alle Detektionseinheiten erreicht. Dadurch würde die Detektions-Anordnung mehr Zeit benötigen, bis sie ein zuverlässiges Messergebnis liefert. Außerdem wäre in manchen Fällen der Fluss von Gas zu den Detektionseinheiten erschwert, was zu fehlerhafte Messergebnissen führen kann.

Erfindungsgemäß umfasst die Detektions-Anordnung zwei Detektor-Kompensator-Paare. In einer bevorzugten Ausgestaltung umfasst die Detektions-Anordnung zusätzlich ein drittes Detektor-Kompensator-Paar, welches ebenfalls genau einen Detektor und genau einen Kompensator umfasst. Insgesamt umfasst die Detektions-Anordnung gemäß dieser Ausgestaltung mindestens drei Detektoren und mindestens einen Kompensator.

Das erste Detektor-Kompensator-Paar liefert einen ersten Zielgas-Konzentrations-Schätzwert. Das zweite Paar liefert einen zweiten Zielgas-Konzentrations-Schätzwert. Die Auswerteeinheit vermag diese beiden Schätzwerte miteinander zu vergleichen. Das dritte Paar bleibt bevorzugt ausgeschaltet, wenn diese beiden Schätzwerte sich um nicht mehr als eine vorgegebene Schranke voneinander unterscheiden. Wie groß diese Schranke ist, kann davon abhängen, welche Zeit zwischen dem Zustand, in dem das erste Paar eingeschaltet und das zweite Paar ausgeschaltet ist, und dem Zustand, in dem das zweite Paar eingeschaltet und das erste Paar ausgeschaltet ist, verstreicht. Falls die beiden Schätzwerte sich um mehr als diese Schranke voneinander unterscheiden, so bewirkt die Gasdetektionsvorrichtung, dass das dritte Paar eingeschaltet wird.

Das dritte Paar steht also in Reserve und wird nicht eingeschaltet und daher nicht verwendet, solange die beiden Schätzwerte vom ersten Paar und vom zweiten Paar sich jeweils um nicht mehr als die Schranke voneinander unterscheiden. So lange wird das dritte Paar daher auch nicht vergiftet. Bei einem Unterschied unterhalb der Schranke sind sowohl das erste Paar als auch das zweite Paar intakt. Das dritte Paar wird nicht vergiftet, solange es ausgeschaltet bleibt. Falls die beiden Schätzwerte um mehr als die Schranke voneinander abweichen, ist dies ein Indiz dafür, dass der stärker belastete Detektor des ersten und / oder des zweiten Paars stark vergiftet ist. In dieser Situation wird wenigstens zeitweise das dritte Paar verwendet. In vielen Fällen lässt sich anhand des gemessenen Werts der Detektionsgröße des dritten Paars entscheiden, ob der Detektionsgrößen-Wert vom ersten Paar oder der Detektionsgrößen-Wert vom zweiten Paar korrekt ist und daher das Paar, das einen vermutlich inkorrekten Detektionsgrößen-Wert liefert, abgeschaltet und / oder überprüft werden muss. Beispielsweise lassen das erste Paar und / oder das zweite Paar sich überprüfen oder sogar erneut justieren / kalibrieren.

Jeder Detektor dieser mindestens drei Paare weist jeweils eine wirksame Oberfläche auf. Diese wirksame Oberfläche kommt in Kontakt mit einer Gasprobe. Der Detektor vermag mittels der wirksamen Oberfläche ein Zielgas in dieser Gasprobe zu oxidieren - natürlich nur dann, wenn die Gasprobe mindestens ein oxidierbares Zielgas umfasst. Bevorzugt weist der Detektor des dritten Paars eine größere wirksame Oberfläche auf als der Detektor des ersten Paares und als der Detektor des zweiten Paars. In vielen Fällen vermag der Detektor des dritten Paars dadurch einen Messwert mit einer besonders großen Zuverlässigkeit zu liefern.

In einer Ausgestaltung wird für jedes Detektor-Kompensator-Paar jeweils ein Nullwert vorgegeben. Der Nullwert wird beispielsweise in einer vorhergehenden Kalibrierung festgelegt oder ermittelt. Die Detektionsgröße des Paars nimmt diesen Nullwert dann an, wenn kein Zielgas in der Umgebung des Paars vorhanden ist. In vielen Fällen nimmt die Detektionsgröße eines Paars auch dann nicht den Wert Null an, wenn kein Zielgas in der Umgebung vorhanden ist. Mit anderen Worten: Der Nullwert ist in der Regel ungleich Null. Ein üblicher Grund ist der folgende: Die Nullwerte des Detektors und des Kompensators eines Paares unterscheiden sich voneinander, insbesondere aufgrund von konstruktionsbedingten und / oder fertigungsbedingten Unterschieden. Dank des Nullwerts ist es nicht erforderlich, diese Unterschiede vorab auf Null zu bringen.

Ein eingeschaltetes Detektor-Kompensator-Paar liefert einen Messwert für die Detektionsgröße. Als Detektionsgrößen-Wert verwendet die Auswerteeinheit bevorzugt die Differenz aus dem Messwert und dem vorgegebenen, beispielsweise abgespeicherten, Nullwert dieses Paars. Die Auswerteeinheit entscheidet über das Vorhandensein des Zielgases und / oder misst die Zielgas-Konzentration abhängig von dieser Differenz. Die Auswerteeinheit vermag in einer Ausgestaltung für jede Detektionseinheit die Differenz zwischen dem Wert, den die Detektionsgröße aktuell annimmt, und dem Nullwert der Detektionsgröße zu berechnen. Dadurch liegen für jedes eingeschaltete Paar zwei Differenzen vor. Möglich ist auch, einen Nullwert für eine Brückenschaltung vorzugeben.

Diese Ausgestaltung mit den Nullwerten erspart die Notwendigkeit, die Detektionseinheiten dergestalt baugleich zu realisieren und zu fertigen, dass die Detektionsgröße für jede Detektionseinheit bei Abwesenheit von Zielgas und bei gleichen Umgebungsbedingungen denselben Wert annimmt. Vielmehr kompensieren die Nullwerte bis zu einem gewissen Grad bauartbedingte Unterschiede.

In einer Ausgestaltung umfasst die Gasdetektionsvorrichtung ein Gehäuse, welches mindestens die Detektions-Anordnung und die Messeinheit aufnimmt, optional eine eigene Spannungsversorgungseinheit. Die Auswerteeinheit kann sich ebenfalls innerhalb dieses Gehäuses befinden. Möglich ist auch, dass die Auswerteeinheit räumlich vom Gehäuse entfernt und mit der Messeinheit über eine Datenverbindung, insbesondere über eine Datenverbindung per Funkwellen oder per Kabel, verbunden ist. Diese Ausgestaltung erspart die Notwendigkeit, die Auswerteeinheit innerhalb des Gehäuses anzuordnen. Möglich ist, dass dieselbe Auswerteeinheit mit unterschiedlichen Gasdetektionsvorrichtungen verbunden ist.

In einer Ausgestaltung umfasst die Gasdetektionsvorrichtung eine eigene Spannungsversorgungseinheit. In einer anderen Ausgestaltung lässt sie sich mit einem stationären Spannungsversorgungsnetz verbinden.

In einer Ausgestaltung umfasst die Gasdetektionsvorrichtung eine Ausgabeeinheit, die ein Messergebnis in mindestens einer von einem Menschen wahrnehmbaren Form ausgibt, insbesondere visuell, akustisch und / oder haptisch (durch Vibrationen). In einer anderen Ausgestaltung umfasst die Gasdetektionsvorrichtung eine Kommunikationseinheit, die eine Nachricht mit einem Messergebnis an einen räumlich entfernten Empfänger zu übermitteln vermag. Diese beiden Ausgestaltungen lassen sich miteinander kombinieren.

Im Folgenden wird die Erfindung anhand eines Ausführungsbeispiels beschrieben. Hierbei zeigt
- Figur 1: eine Gasdetektionsvorrichtung mit einem Detektor und einem Kompensator, die beide als Pellistoren ausgestaltet sind, wobei die Brückenspannung gemessen wird;
- Figur 2: in einer perspektivischen Darstellung eine flache Detektionseinheit;
- Figur 3: die Detektionseinheit von Figur 3 in einer Draufsicht;
- Figur 4: eine Gasdetektionsvorrichtung mit einem Detektor, der als Pellistor ausgestaltet ist, und einem flachen Kompensator, wobei sowohl die Detektor-Spannung als auch die Kompensator-Spannung gemessen werden;
- Figur 5: eine Draufsicht auf eine erfindungsgemäße Gasdetektionsvorrichtung mit neun Detektoren und einem Kompensator, also acht Paaren, auf einer Platine;
- Figur 6: einen beispielhaften zeitlichen Verlauf eines Selbsttests der Gasdetektionsvorrichtung nach der Detektion eines steilen Anstiegs der Zielgas-Konzentration, wobei alle Paare intakt sind;
- Figur 7: eine Abwandlung des zeitlichen Verlaufs von Figur 6, wobei ein Paar defekt ist;
- Figur 8: einen beispielhaften zeitlichen Verlauf eines Selbsttests der Gasdetektionsvorrichtung nach der Detektion eines steilen Abfalls der Zielgas-Konzentration, wobei alle Paare intakt sind;
- Figur 9: eine Abwandlung des Verlaufs von Figur 8, wobei ein Paar defekt ist.

Die erfindungsgemäße Gasdetektionsvorrichtung wird dafür verwendet, um mindestens ein brennbares Gas zu detektieren und / oder um die Konzentration des brennbaren Gases zu ermitteln. Dieses brennbare Gas wird im Folgenden als "Zielgas" bezeichnet. In einer Ausgestaltung wird dann, wenn mehrere Zielgase vorhanden sind, die Summe der Zielgas-Konzentrationen ermittelt.

In einer Anwendung wird die Gasdetektionsvorrichtung dafür verwendet, um einen räumlichen Bereich zu überwachen und hierbei mindestens ein brennbares Zielgas zu detektieren und / oder um die Zielgas-Konzentration zu ermitteln oder um sicherzustellen, dass kein brennbares Zielgas oberhalb einer Nachweisgrenze vorhanden ist. Der räumliche Bereich ist beispielsweise eine Raffinerie oder eine sonstige Produktionsanlage, das Innere eines Gebäudes, ein Bergwerk, das Innere eines Fahrzeugs oder das Innere eines Flugzeugs. Das brennbare Gas ist beispielsweise Methan (CH₄).

In einer anderen Anwendung wird die Gasdetektionsvorrichtung dafür verwendet, um einen Probanden auf eine Substanz zu untersuchen, die sich in ausgeatmeter Luft feststellen lässt, insbesondere auf Alkohol. Bekanntlich enthält die Luft, die ein Proband ausatmet, Atemalkohol, vorausgesetzt dieser Proband hat Alkohol zu sich genommen und dieser Alkohol ist in seinem Körper noch nicht vollständig abgebaut. In diesem Fall ist im Blut des Probanden und / oder in seiner Lunge und in seinem Mund noch Alkohol vorhanden. Bei dieser Anwendung gibt der Proband eine Atemprobe in ein Mundstück der Gasdetektionsvorrichtung ein. Wenigstens ein Teil der abgegebenen Atemprobe gelangt in das Innere der Gasdetektionsvorrichtung. Bei dieser Anwendung ist gasförmiger Atemalkohol das brennbare Zielgas.

In einer Realisierungsform ist die Gasdetektionsvorrichtung ein tragbares Gerät, welches ein Mensch in einer Hand halten oder auch an seiner Kleidung oder Schutzausrüstung befestigen kann. Beispielsweise führt ein Benutzer eine solche Gasdetektionsvorrichtung mit sich, während er sich in einem Bereich aufhält, in dem mindestens ein brennbares Zielgas vorhanden sein kann. Oder einem Probanden wird die Gasdetektionsvorrichtung vor den Mund gehalten, damit der Proband eine Atemprobe eingibt.

Bevorzugt erzeugt die Gasdetektionsvorrichtung bei der ersten Anwendung, optional auch bei der zweiten Anwendung, einen Alarm in mindestens einer Form, die ein Mensch wahrnehmen kann, wenn eine Zielgas-Konzentration oberhalb einer vorgegebenen Schranke auftritt. Der Alarm wird beispielsweise visuell, akustisch und oder haptisch (durch Vibrationen) ausgegeben. Die Gasdetektionsvorrichtung umfasst bei diesen Anwendungen eine eigene Spannungsversorgungseinheit.

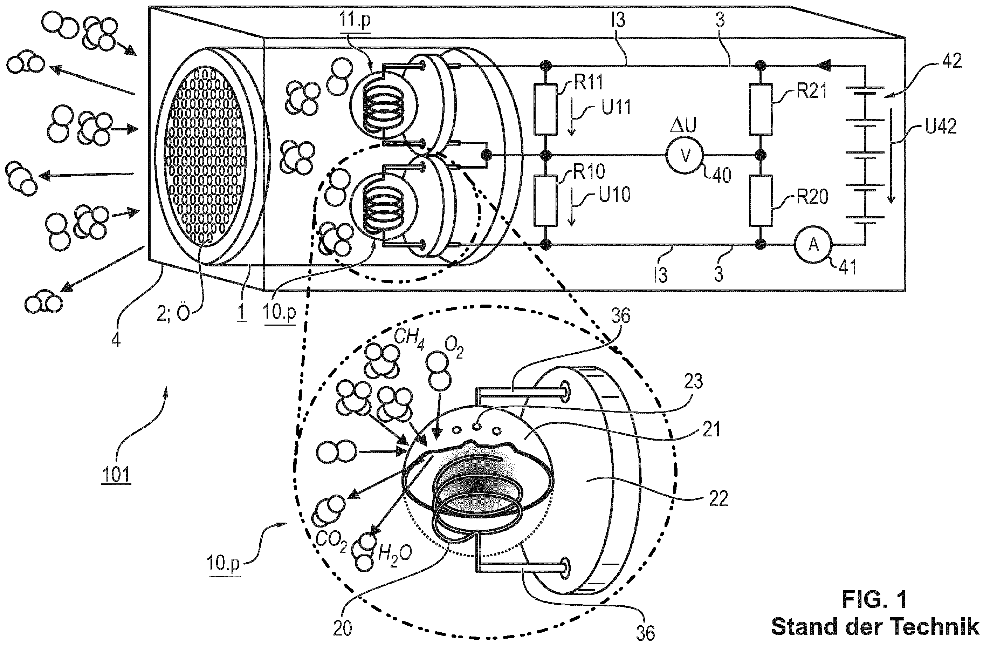

Figur 1 zeigt beispielhaft eine Gasdetektionsvorrichtung 100, wie sie aus dem Stand der Technik bekannt ist. Das nachfolgend mit Bezug auf Figur 1 beschriebene Prinzip lässt sich auch für eine erfindungsgemäße Gasdetektionsvorrichtung anwenden. Das Prinzip ist unter den Bezeichnungen "Wärmetönungs-Sensor" und "katalytischer Sensor" bekannt geworden.

In der in Figur 1 gezeigten Realisierungsform umgibt ein äußeres Gehäuse 4, beispielsweise aus einem harten Kunststoff, ein hitzebeständiges inneres zylinderförmiges Gehäuse 1, beispielsweise aus Stahl. Eine Gasprobe kann durch eine Öffnung Ö mit einem Flammschutz 2 in das Innere des inneren Gehäuses 1 gelangen, beispielsweise indem die Gasprobe angesaugt wird oder durch Diffusion.

Die Gasdetektionsvorrichtung 100 umfasst einen Detektor 10.p und einen Kompensator 11.p. Nachfolgend wird für einen Detektor und einen Kompensator der Oberbegriff "Detektionseinheit" verwendet. Derjenige Bestandteil der Gasdetektionsvorrichtung 100, der den oder jeden Detektor sowie den oder jeden Kompensator und die elektrischen Kontaktierungen umfasst, wird als "Detektions-Anordnung" DA bezeichnet. Bevorzugt befindet die Detektions-Anordnung DA sich vollständig im Inneren des inneren Gehäuses 1.

Der Detektor 10.p umfasst ein elektrisch leitendes Bauteil mit einem heizenden Segment 20 und mit zwei elektrischen Kontaktierungen 36, wobei die beiden elektrischen Kontierungen 36 auf einer Platte 22 montiert sind. Das heizende Segment 20 hat in der gezeigten Realisierungsform die Form eines spiralförmigen Drahts und ist bevorzugt aus einem Material, welches idealerweise chemisch nicht mit einem zu detektierenden Zielgas reagiert, also inert ist. Eine Keramikummantelung 21 umgibt das heizende Segment 20 und hat im gezeigten Beispiel die Form einer Vollkugel. Auf die äußere Oberfläche der Keramikummantelung 21 ist eine katalytische Beschichtung aufgebracht, welche durch Kreise 23 angedeutet ist. Als katalytisches Material wird beispielsweise Platin oder Palladium oder ein anderes geeignetes Metall oder eine andere Legierung verwendet. Bevorzugt ist die Keramikummantelung 21 porös, so dass die Gasprobe auch in das Innere der Keramikummantelung 21 gelangen kann.

Wenn an die elektrischen Kontaktierungen 36 eine elektrische Spannung angelegt wird, so fließt ein Strom durch das heizende Segment 20, und das heizende Segment 20 erhitzt sich. Das erhitzte heizende Segment 20 oxidiert ein brennbares Zielgas im Inneren des inneren Gehäuses 1, und zwar in Zusammenwirken mit dem katalytische Material 23 in der Keramikummantelung 21. Beispielhaft wird angedeutet, dass beim Oxidieren Methan in Kohlendioxid und Wasser umgewandelt wird, dass also die chemische Reaktion

CH₄ + 2O₂ → 2H₂O + CO₂

stattfindet. Bei dieser chemischen Reaktion wird Wärmeenergie freigesetzt. Diese freigesetzte Wärmeenergie erhitzt weiter die Temperatur des heizenden Segments 20. Die Temperatur des heizenden Segments 20 ist daher ein Maß für die Konzentration von Zielgas im Inneren des Gehäuses 1.

Ein Maß für die Temperatur des heizenden Segments 20 wird gemessen. In einer Ausgestaltung wird der elektrische Widerstand des heizenden Segments 20 gemessen. Bekanntlich verändert sich bei vielen elektrisch leitenden Materialien der elektrische Widerstand mit der Temperatur. In der Regel ist der elektrische Widerstand umso größer, je größer die Temperatur ist.

Die Temperatur des heizenden Segments 20 wird aber nicht nur von der Zielgas-Konzentration beeinflusst, sondern auch von Umgebungsbedingungen, insbesondere von der Umgebungstemperatur und / oder von der Temperatur der Gasprobe im Inneren des inneren Gehäuses 1. Daher ist im Inneren des Gehäuses 1 zusätzlich der Kompensator 11.p angeordnet. Der Kompensator 11.p umfasst ebenfalls ein heizendes Segment sowie eine Keramikummantelung, jedoch im Ausführungsbeispiel keine katalytische Beschichtung und in einer anderen möglichen Realisierungsformen eine geringere katalytische Beschichtung. Daher vermag der Kompensator 11.p gar kein brennbares Zielgas zu oxidieren oder dies wenigstens langsamer oder weniger zu tun. Die Umgebungsbedingungen wirken hingegen idealerweise genauso auf den Kompensator 11.p wie auf den Detektor 10.p. Daher ermöglicht es der Kompensator 11.p, den Einfluss von Umgebungsbedingungen auf den Detektor 10.p bis zu einem gewissen Grad rechnerisch zu kompensieren.

In der Realisierungsform gemäß Figur 1 umfasst die Gasdetektionsvorrichtung 100 zusätzlich folgende Bestandteile:
- eine Spannungsversorgungseinheit 42, beispielsweise einen Satz von wiederaufladbaren Batterien (Akkumulatoren),
- einen elektrischen Widerstand R20, der parallel zum Detektor 10.p geschaltet ist,
- einen elektrischen Widerstand R21, der parallel zum Kompensator 11.p geschaltet ist,
- eine elektrische Leitung 3,
- einen Spannungs-Sensor 40 und
- einen Stromstärken-Sensor 41.

In Figur 1 werden außerdem folgende elektrischen Kenngrößen gezeigt:
- der elektrische Widerstand R10 des Detektors 10.p,
- der elektrische Widerstand R11 des Kompensators 11.p,
- die Spannung U42 an der Spannungsversorgungseinheit 42,
- die Stärke I3 des Stroms, der durch die elektrische Leitung 3 fließt,
- die elektrische Spannung U10, die am Detektor 10.p anliegt, und
- die elektrische Spannung U11, die am Kompensator 11.p anliegt.

Anmerkung: Der Begriff "elektrischer Widerstand" bezeichnet zum einen ein Bauteil und zum anderen eine elektrische Eigenschaft eines Bauteils.

In der Ausführungsbeispiel gemäß Figur 1 bilden die beiden Detektionseinheiten der Detektions-Anordnung eine Wheatstone'sche Messbrücke. Der Detektor 10.p und der Kompensator 11.p sind in Reihe geschaltet. Der elektrische Widerstand des Spannungs-Sensors 40 ist hoch im Vergleich zu den elektrischen Widerständen der Bauteile 10.p, 11.p, R20 und R21. In einer Ausgestaltung misst der Spannungs-Sensor 40 direkt die sogenannte Brückenspannung ΔU = (U10 - U11) / 2. Der Stromstärken-Sensor 41 misst die Stromstärke I3.

Idealerweise ist die Brückenspannung ΔU gleich Null, wenn kein brennbares Zielgas im Inneren des Gehäuses 1 vorhanden ist. Insbesondere aufgrund von bauartbedingten Unterschieden ist in der Regel die Brückenspannung ΔU aber ungleich Null, auch wenn kein brennbares Zielgas vorliegt. Daher wird vorab ein sogenannter Nullpunkt ΔU,0 ermittelt, bevorzugt empirisch ermittelt. Der Nullpunkt ΔU,0 ist die Brückenspannung ΔU beim Ausbleiben von brennbarem Zielgas.

Eine gemessene Detektionsgröße korreliert mit der Konzentration von brennbarem Zielgas. Eine in Figur 1 nicht gezeigte signalverarbeitende Auswerteeinheit wendet einen vorgegebenen funktionalen Zusammenhang auf die Detektionsgröße ΔU - ΔU,0 an und leitet einen Schätzwert für die Zielgas-Konzentration her.

In einer Realisierungsform wird die Stromstärke I3 durch eine Regelung (closed-loop control) konstant gehalten. Die Spannung ΔU korreliert dann mit dem elektrischen Widerstand und daher mit der Temperatur-Änderung, die durch die Zielgas Oxidierung bewirkt wird. Die Differenz ΔU - ΔU,0 fungiert als die Detektionsgröße.

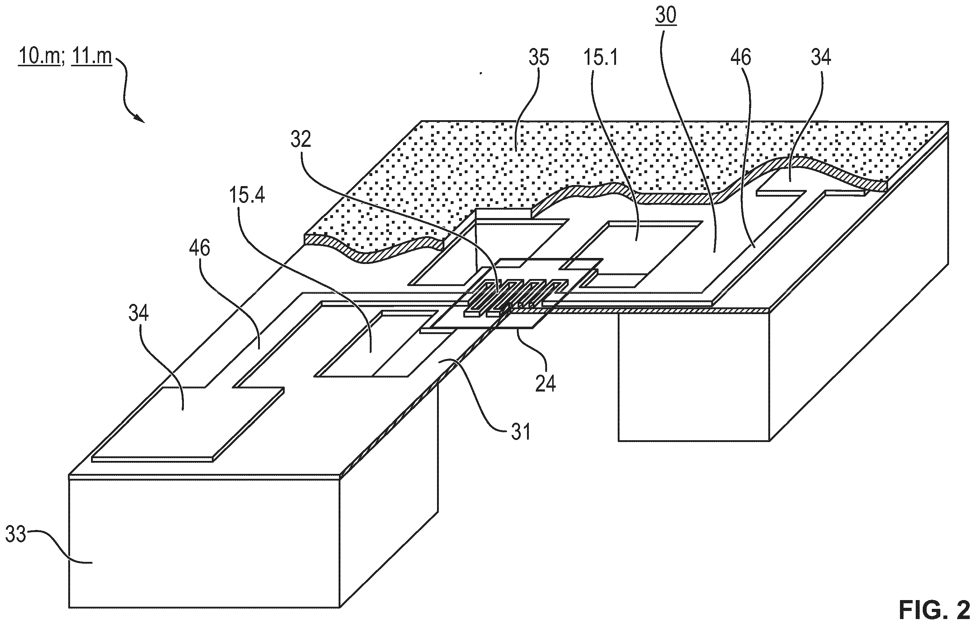

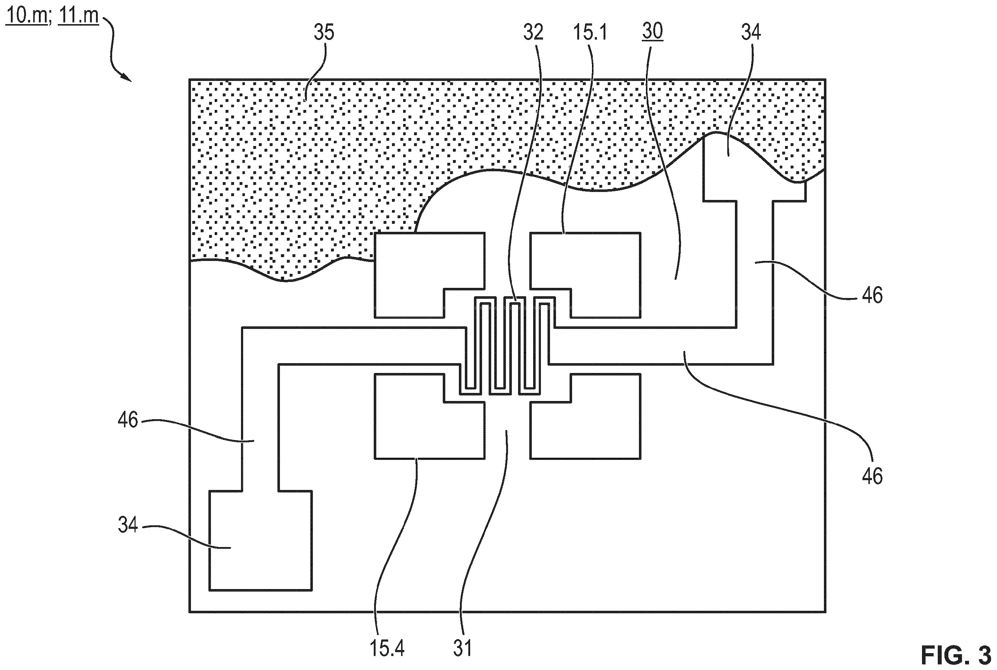

Figur 2 zeigt in einer perspektivischen Darstellung eine andere Realisierungsform des Detektors und des Kompensators. Der Detektor gemäß dieser Realisierungsform wird mit 10.m bezeichnet, der Kompensator mit 11.m. Figur 3 zeigt den Detektor 10.m und den Kompensator 11.m von Figur 2 in einer Draufsicht. Der Detektor 10.m wendet das gleiche Prinzip an wie der Detektor 10.p von Figur 1, ist aber wesentlich flacher als dieser. Das entsprechende gilt für den Kompensator 11.m.

Der Detektor 10.m und der Kompensator 11.m umfassen jeweils folgende Bestandteile:
- ein elektrisch leitendes Bauteil 30 mit einem heizenden Segment 32 und einer elektrischen Kontaktierung 46, wobei das Bauteil 30 die Form einer Leiterbahn aufweist,
- eine Schutzschicht 35,
- eine Trägerplatte 31, die sich in einer Ebene erstreckt, wobei diese Ebene schräg auf der Zeichenebene von Figur 2 steht und in der Zeichenebene von Figur 3 liegt,
- ein Wafer-Substrat 33, welches die Trägerplatte 31 trägt, und
- elektrische Kontaktstellen 34 für das elektrisch leitende Bauteil 30.

Die Schutzschicht 35 überdeckt wenigstens die Leiterbahn 30, bevorzugt die gesamte Trägerplatte 31, und verhindert, dass die Leiterbahn 30 in direkten Kontakt mit einer Gasprobe kommt. **In** einer Realisierungsform ist die Schutzschicht 35 aus Siliziumnitrid hergestellt. Beim Detektor 10.m ist auf die Schutzschicht 35 katalytisch wirksames Material aufgebracht, und zwar wenigstens in einem Bereich 24 oberhalb des heizenden Segments 32.

Der Kompensator 11.m umfasst die gleichen Bestandteile wie der Detektor 10.m. Bevorzugt unterscheidet sich der Kompensator 11.m vom Detektor 10.m dadurch, dass auf die Schutzschicht 35 des Kompensators 11.m weniger oder sogar überhaupt kein katalytisches Material eingelassen ist, so dass der Katalysator 11.m kein brennbares Zielgas zu oxidieren vermag oder dies in geringerem Umfang als der Detektor 10.m zu tun vermag.

Die Leiterbahn 30 kann aus dem gleichen elektrisch leitenden Material hergestellt sein wie das Bauteil 20 des Detektors 10.p von Figur 1. Das heizende Segment 32 der Leiterbahn 30 wird bevorzugt dadurch realisiert, dass die Leiterbahn 30 im Bereich 24 zickzack-förmig oder auf andere Weise gebogen oder gewellt ist. Alternativ weist die Leiterbahn 30 einen Querschnitt auf, der über die Länge der Leiterbahn 30 variiert, so dass dann, wenn Strom durch die Leiterbahn 30 fließt, eine ausreichend hohe Arbeitstemperatur erzielt wird. Bevorzugt betragen die maximale Abmessung der Leiterbahn 30 und damit die maximale Abmessung des heizenden Segments 32 in der Ebene der Trägerplatte 31 weniger als 2 mm, besonders bevorzugt weniger als 1 mm, besonders bevorzugt zwischen 0,1 mm und 0,5 mm. Das heizende Segment 32 oder auch die gesamte Leiterbahn 30 ist aus einem Metall hergestellt, beispielsweise aus Platin oder Wolfram oder Tungsten oder Titan, oder aus einer Legierung, die mindestens eine der vorgenannten Metalle umfasst.

Die Trägerplatte 31 hat bevorzugt eine Dicke von weniger als 10 µm, besonders bevorzugt weniger als 2 µm, insbesondere eine Dicke von höchstens 1 µm, und ist bevorzugt aus einem Material hergestellt, welches Silizium enthält, beispielsweise aus einem glasähnlichen Material. Die Trägerplatte 31 ist im Ausführungsbeispiel mithilfe von vier Stegen an dem Wafer-Substrat 33 befestigt. Bei der Herstellung vermag ein Greifer den Kompensator 11.1 am Wafer-Substrat 33 zu greifen und zu montieren.

Der elektrisch leitende Leiterbahn 30 ist auf eine Oberfläche der Trägerplatte 31 aufgetragen und bevorzugt in diese eingebettet. Bevorzugt hat die Leiterbahn 30 eine maximale Dicke, also eine maximale Abmessung senkrecht zur Ebene der Trägerplatte 31, von maximal 1 µm, besonders bevorzugt von maximal 0,5 µm. Die Trägerplatte 31 entkoppelt die Leiterbahn 30 thermisch und bevorzugt auch elektrisch von der Umgebung. Die Trägerplatte 31 steht auf mindestens einer Seite in Kontakt mit der umgebenden Luft, was eine gute thermische Isolierung der Leiterbahn 30 bewirkt. Die Trägerplatte 31 kann Aussparungen 15.1, ... enthalten. Die Aussparungen 15.1, ... können zu einer streifenförmigen Trägerplatte 31 führen. Die Trägerplatte 31 kann auch vollflächig ausgebildet sein.

In einer Realisierungsform ist das Wafer-Substrat 33 weniger als 1 mm dick, besonders bevorzugt weniger als 0,4 mm dick, und hat einen maximalen Durchmesser von mehreren Millimetern. Die Trägerplatte 31 wird auf das Wafer-Substrat 33 aufgebracht, beispielsweise durch eine chemische Gasphasenabscheidung oder durch Aufdampfen. Bevorzugt ist in denjenigen Bereich des Wafer-Substrats 33, welches sich unterhalb des heizenden Segments 32 befindet, eine Aussparung eingelassen, sodass das heizende Segment 32 an zwei Seiten von Luft umgeben ist. Dies verbessert die thermische Isolierung. Die Aussparung wird beispielsweise hergestellt, indem sie in das Material eingeätzt wird.

Die elektrische Verbindung 46 verbindet das heizende Segment 32 mit den elektrischen Kontaktstellen 34 auf der Trägerplatte 31. Die Schutzschicht 35 isoliert die Leiterbahn 30 und damit das heizende Segment 32 elektrisch von der Umgebung und reduziert die Gefahr einer Beschädigung. Insbesondere verhindert die Schutzschicht 35, dass die Leiterbahn 30 mit einem Gas aus der Umgebung in Kontakt kommt, vor allem mit einem für die Leiterbahn 30 potenziell schädigenden Gas, beispielsweise Wasserstoff.

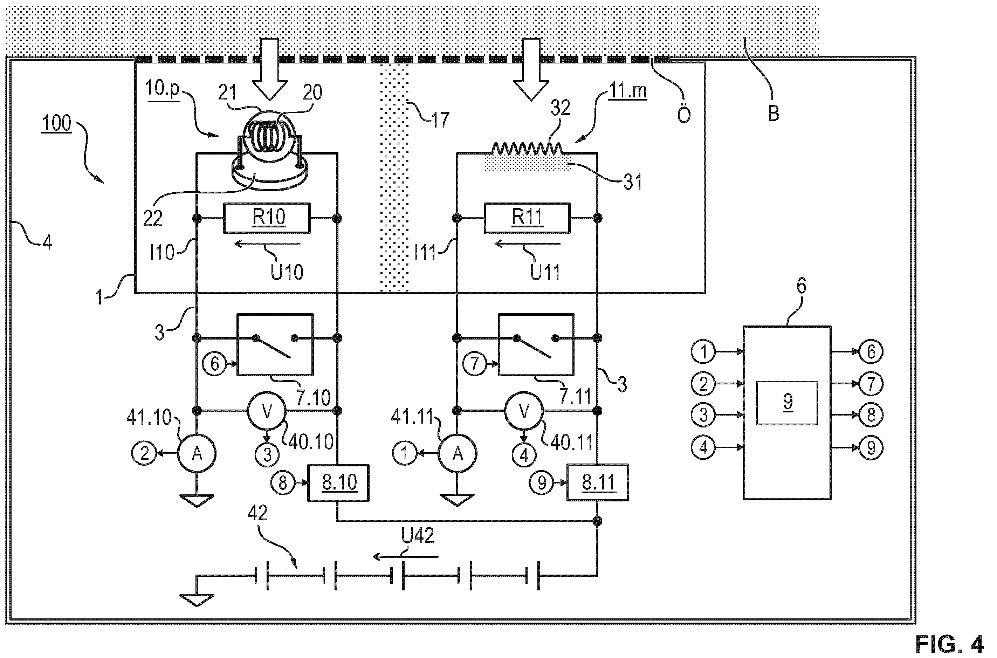

Figur 4 zeigt schematisch eine Ausgestaltung, bei der der Detektor so wie der Detektor 10.p in Figur 1 ausgestaltet ist, während der Kompensator so wie der Kompensator 11.m in Figur 2 und Figur 3 ausgestaltet ist.

Die Gasdetektionsvorrichtung 100 von Figur 4 umfasst folgende Bestandteile:
- den Detektor 10.p mit dem heizenden Segment 20, der Keramikummantelung 21 und der Beschichtung 23 aus einem katalytischen Material,
- den Kompensator 11.m mit einem heizenden Segment 32 und einer Trägerplatte 31 und optional ebenfalls katalytischem Material,
- die Spannungsquelle 42,
- zwei Schalter 7.10 und 7.11,
- einen ansteuerbaren Spannungs-Aktor 8.10, der die elektrische Spannung U10, welche am Detektor 10.p anliegt, zu verändern vermag,
- einen ansteuerbaren Spannungs-Aktor 8.11, der die elektrische Spannung U11, welche am Kompensator 11.m anliegt, zu verändern vermag,
- zwei Spannungs-Sensoren 40.10 und 40.11,
- zwei Stromstärken-Sensoren 41.10 und 41.11,
- die elektrischen Widerstände R20 und R21, vgl. Figur 1,
- ein schematisch gezeigtes signalverarbeitendes Steuergerät 6 mit einer Auswerteeinheit 9,
- eine optionale thermische Barriere 17 zwischen dem Detektor 10.p und dem Kompensator 11.m,
- das äußere Gehäuse 4 mit der Öffnung Ö und
- das stabile innere Gehäuse 1.

Die Ausgestaltung gemäß Figur 4 lässt sich auch mit einem Detektor 10 realisieren, der so wie der Detektor 10.m von Figur 2 und Figur 3 ausgestaltet ist. Möglich ist auch, dass der Kompensator 11 so wie der Kompensator 11.p von Figur 1 ausgestaltet ist.

Angedeutet sind weiterhin der zu überwachende räumliche Bereich B sowie Pfeile, die veranschaulichen, wie eine Gasprobe aus dem Bereich B durch die Öffnung Ö in das Innere des Gehäuses 1 fließt. Der Flammschutz 2 wird nicht gezeigt.

Der Spannungs-Sensor 40.10 misst die elektrische Spannung U10, die am Detektor 10.p anliegt, der Spannungs-Sensor 40.11 die am Kompensator 11.m anliegende Spannung U11. Im Gegensatz zur Ausgestaltung gemäß Figur 1 können durch den Detektor 10.p und den Kompensator 11.m Ströme von zwei unterschiedlichen Stromstärken fließen. Die beiden Stromstärken-Sensoren 41.10 und 41.11 messen diese beiden Stromstärken I10 bzw. I11.

Bevorzugt werden durch jeweils eine Regelung (closed-loop control) die beiden Stromstärken I10 und I11 konstant gehalten. Die Temperatur des heizenden Segments 20 des Detektors 10.p korreliert mit dem elektrischen Widerstand, und bei konstanter Stromstärke I10 korreliert der elektrische Widerstand mit der Spannung U10. Entsprechend korrelieren die Temperatur und der elektrische Widerstand des heizenden Segments 32 des Kompensators 11.m mit der Spannung U11. Bevorzugt werden vorab zwei Nullwerte für die Spannungen ermittelt, nämlich ein Nullwert U10,0 für die Spannung U10 am Detektor 10.p und ein Nullwert U11,0 für die Spannung U11 am Kompensator 11.m bei Abwesenheit von brennbarem Zielgas. Bevorzugt wird als Detektionsgröße die Differenz (U10-U10,0) - (U11 - U11,0) verwendet.

Um elektrische Energie einzusparen, bewirkt im Ausführungsbeispiel das Steuergerät 6, dass am Detektor 10.p und am Kompensator 11.m nicht dauerhaft eine elektrische Spannung anliegt, sondern eine gepulste Spannung. Das Steuergerät 6 steuert die beiden Schalter 7.10 und 7.11 mit dem Ziel an, die am Detektor 10.p bzw. am Kompensator 11.m anliegende elektrische Spannung U10 bzw. U11 zu pulsen. Bevorzugt lassen sich die Pulsrate und / oder die Pulsdauer der am Detektor 10.p anliegenden elektrischen Spannung U10 unabhängig von der Pulsrate und der Pulsdauer der am Kompensator 11.m anliegenden elektrischen Spannung U11 einstellen und verändern.

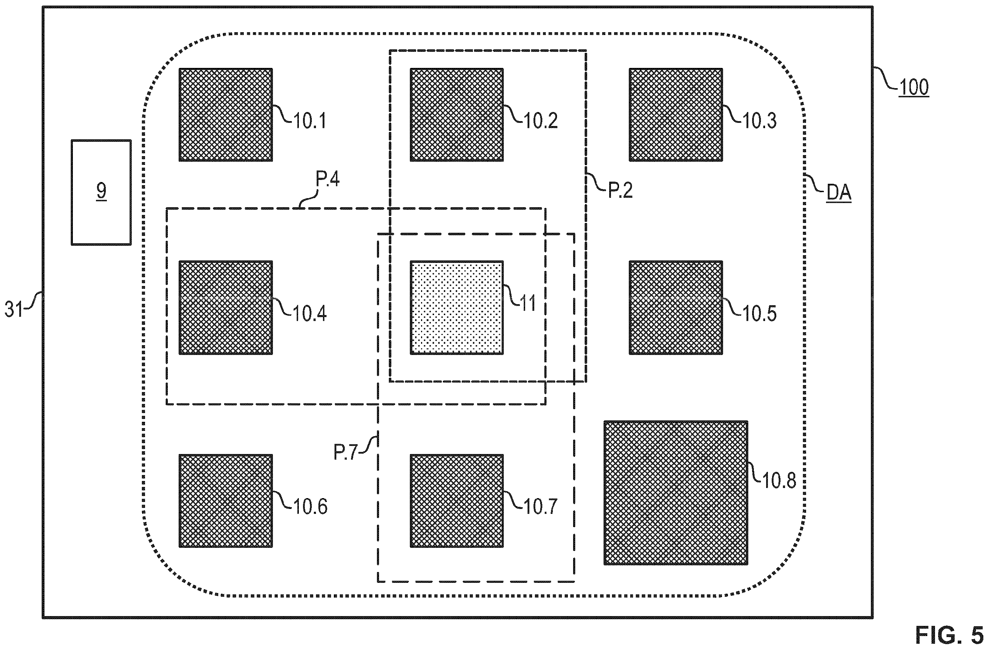

Figur 5 zeigt schematisch in einer Draufsicht eine erfindungsgemäße Gasdetektionsvorrichtung 100. Auf eine Trägerplatte 31, die als Platine ausgestaltet ist und sich in der Zeichenebene von Figur 5 erstreckt, sind neun schematisch gezeigte Detektionseinheiten aufgebracht, nämlich acht Detektoren 10.1, ..., 10.8 und ein Kompensator 11, außerdem das Steuergerät 9. Bevorzugt sind jeder Detektor 10.1, ..., 10.8 sowie der Kompensator 11 so aufgebaut wie mit Bezug auf Figur 2 und Figur 3 beschrieben, sind also flache Bauteile. Möglich ist auch, dass mindestens ein Detektor 10.1, ..., 10.8 oder der Kompensator 11 so wie mit Bezug auf Figur 1 beschrieben aufgebaut ist. Die Trägerplatte 31 stellt die elektrischen Kontaktierungen für die neun Detektionseinheiten 10.1, ..., 10.8, 11 bereit, wobei diese elektrischen Kontaktierungen in Figur 5 nicht gezeigt sind. Die neun Detektionseinheiten 10.1, ..., 10.8, 11 sowie die Platine 31 gehören zur Detektions-Anordnung DA.

Gesehen in die Betrachtungsrichtung von Figur 5 sind die neun Detektoreinheiten 10.1, ..., 10.8, 11 überlappungsfrei nebeneinander auf der Trägerplatte 31 angeordnet, und zwischen zwei benachbarten Detektionseinheiten tritt jeweils ein Abstand auf. Eine Gasprobe kann daher gleichzeitig alle neun Detektoreinheiten erreichen, ohne das eine Detektoreinheit eine andere Detektoreinheit abschattet, also den Vorgang einschränkt, dass die Gasprobe die andere Detektoreinheit erreicht. In der gezeigten Realisierungsform bilden die neun Detektoreinheiten 10.1, ..., 10.8, 11 eine 3x3-Matrix, also eine Anordnung mit drei Zeilen und drei Spalten mit jeweils drei Detektoreinheiten, wobei der einzige Kompensator 11 in der Mitte angeordnet ist. Weil der Kompensator 11 in der Mitte angeordnet ist, tritt zwischen dem Kompensator 11 und jedem Detektor 10.1, ..., 10.8 nur jeweils ein relativ geringer Abstand auf, sodass die Umgebungsbedingungen um den Kompensator 11 und um den Detektor 10.1, ..., 10.8 annähernd übereinstimmen.

Selbstverständlich ist auch eine andere Anzahl von Zeilen und / oder Spalten einer Platine mit Detektoreinheiten sowie eine andere Anzahl von Detektoreinheiten pro Zeile und / oder pro Reihe möglich. Der Kompensator 11 ist bevorzugt, aber nicht notwendigerweise im Zentrum angeordnet. Die Gasdetektionsvorrichtung 100 kann auch mehrere solche Platinen mit Detektoreinheiten umfassen, wobei diese Platinen bevorzugt parallel zueinander angeordnet sind und eine Gasprobe jede Platine erreichen kann.

Möglich ist, dass alle neun Detektoreinheiten 10.1, ..., 10.8, 11 die gleiche wirksame Oberfläche haben, wobei die wirksame Oberfläche in Kontakt mit einer Gasprobe kommt, die im Inneren des Gehäuses 1 angeordnet ist. Im gezeigten Beispiel haben hingegen acht Detektoreinheiten 10.1, ..., 10.7, 11 jeweils die gleiche wirksame Oberfläche. Der Detektor 10.8 hat eine größere wirksame Oberfläche, bevorzugt eine um mindestens die Hälfte größere wirksame Oberfläche, besonders bevorzugt eine doppelt so große wirksame Oberfläche, als die Detektoren 10.1 bis 10.7 und als der Kompensator 11.

In der gezeigten Realisierungsform sind auf der Trägerplatte 31 acht Detektor-Kompensator-Paare P.1, ..., P.8 realisiert, wobei das Detektor-Kompensator-Paar P.x den Detektor 10.x und den Kompensator 11 umfasst (x=1,...,8). Der einzige Kompensator 11 gehört also zu jedem der acht Detektor-Kompensator-Paare P.1, ..., P.8. Allgemein umfasst eine erfindungsgemäße Gasdetektionsvorrichtung 100 m Detektoren und n Kompensatoren, wobei m > n >= 1, und m > 1 gilt. Bevorzugt gilt sogar m >= 2*n, besonders bevorzugt m >= 5*n. Bei m Detektoren und n Kompensatoren werden insgesamt bis zu m*n Paare mit jeweils einem Detektor und einem Kompensator gebildet. Ein Vorteil der bevorzugten Ausgestaltung, dass mehr Detektoren als Kompensatoren vorhanden sind, ist der folgende: Auf der erhitzten Oberfläche eines Detektors bilden sich oft Ablagerungen. Der Detektor vergiftet im Laufe des Einsatzes. Auf der erhitzen Oberfläche des Kompensators bilden sich in der Regel deutlich weniger Ablagerungen. Wenn mehr Detektoren als Kompensatoren vorhanden sind, lässt sich in vielen Fällen die Gasdetektionsvorrichtung länger betreiben, auch wenn einzelne Detektoren ausgefallen sind.

Das Steuergerät 9, das in Figur 5 schematisch gezeigt wird, vermag jedes Detektor-Kompensator-Paar P.x unabhängig von jedem anderen Detektor-Kompensator-Paar P.y (y#x) einzuschalten und auszuschalten. Das Steuergerät 9 bewirkt, dass zu jedem Zeitpunkt höchstens ein Paar P.x eingeschaltet ist und dauerhaft oder gepulst mit elektrischer Spannung versorgt wird, während alle anderen Paare P.y ausgeschaltet sind. Falls das Detektor-Kompensator-Paar P.x eingeschaltet ist, liegt sowohl am Detektor 10.x als auch am Kompensator 11 jeweils eine elektrische Spannung an, bevorzugt eine gepulste Spannung, und ein Strom fließt sowohl durch das heizende Segment des Detektors 10.x als auch durch das heizende Segment des Kompensators 11. Der Detektor 10.a und der Kompensator 11 können insbesondere als Wheatstone'sche Messbrücke, wie sie in Figur 1 gezeigt wird, oder parallel zueinander, so wie es in Figur 4 gezeigt ist, geschaltet sein. Vorzugsweise liegt bei eingeschaltetem Paar P.x die elektrische Spannung gepulst am Detektor 10.a und am Kompensator 11 an, um Energie zu sparen.

Erfindungsgemäß wird die Gasdetektionsvorrichtung 100 so betrieben, dass während eines Einsatzes meistens genau ein Paar P.x eingeschaltet ist und die übrigen Paare ausgeschaltet sind. "Meistens" bedeutet, dass zwischen der Aktivierungs-Zeitspanne eines Paars P.x und einer nachfolgenden Aktivierungs-Zeitspanne eines anderen Paars P.y ein Zwischen-Zeitraum verstreicht, in dem alle Paare ausgeschaltet sind, wobei der Zwischen-Zeitraum bevorzugt kürzer als der oder jede Aktivierungs-Zeitspanne ist. Während einer Ruhepause sind in der Regel alle Paare ausgeschaltet. Das eingeschaltete Paar P.x liefert einen Wert für die Detektionsgröße, beispielsweise einen Wert für die Brückenspannung (Ausgestaltung gemäß Figur 1) oder jeweils einen Wert für die Detektor-Spannung und einen Wert für die Kompensator-Spannung (Ausgestaltung gemäß Figur 4). Der Wert für die Detektionsgröße ist ein Maß für die aktuelle Zielgas-Konfiguration. Natürlich ist es möglich, dass verschiedene Paare nacheinander unterschiedliche Werte für die Zielgas-Konzentration liefern, obwohl die Zielgas-Konzentration gleich bleibt. Dies kann außer durch bauart- oder herstellungsbedingte Unterschiede insbesondere durch eine unterschiedliche Vergiftung oder sonstige Alterung eines Paars hervorgerufen werden. Dies wird weiter unten näher erläutert.

Wenn ein Detektor-Kompensator-Paar P.x eingeschaltet ist, so liegt sowohl am Detektor 10.x als auch am Kompensator 11 jeweils eine elektrische Spannung an. In einer ersten Realisierungsform liegt in dem Zeitraum, in dem das Paar P.x eingeschaltet ist, durchgehend sowohl am Detektor 10.x als auch am Kompensator 11 die elektrische Spannung an, und zwar bevorzugt gepulst.

In einer bevorzugten Realisierungsform liegt in dem Zeitraum, in dem das Paar P.x eingeschaltet ist, nur am Detektor 10.x durchgehend die elektrische Spannung an, bevorzugt gepulst, während am Kompensator 11 nur zeitweise eine elektrische Spannung anliegt. Die bevorzugte Realisierungsform lässt sich beispielsweise mithilfe der Schaltung von Figur 4 verwirklichen. Weil der Kompensator 11 weniger oder sogar überhaupt kein Zielgas zu oxidieren vermag, wird dessen Temperatur im Wesentlichen von der anliegenden Spannung und von der Umgebungstemperatur beeinflusst, und die Umgebungstemperatur ändert sich in aller Regel deutlich langsamer als die Konzentration des Zielgases. Daher reicht es in vielen Fällen aus, während einer Aktivierungs-Zeitspanne einmal die am Kompensator 11 anliegende elektrische Spannung oder sonstige Detektionsgröße zu messen und den gemessenen Detektionsgrößen-Wert des Kompensators 11 für die gesamte Aktivierungs-Zeitspanne zu verwenden, in dem das Paar P.x eingeschaltet ist. Die bevorzugte Realisierungsform spart elektrische Energie ein und verlängert die Lebensdauer des Kompensators 11.

Wie bereits dargelegt, lagern sich häufig im Verlauf eines Einsatzes Schadstoffe auf einem erhitzten Detektor 10 ab. Diese Ablagerungen können die Fähigkeit des Detektors 10 einschränken oder sogar völlig unterbinden, brennbares Zielgas zu oxidieren. Der Detektor 10 wird dann zunehmend vergiftet. Ein Detektor-Kompensator-Paar P.x mit einem vergifteten Detektor 10.x vermag häufig nicht mehr zuverlässig ein brennbares Zielgas zu detektieren. Nachfolgend werden Ausgestaltungen beschrieben, wie die erfindungsgemäße Gasdetektionsvorrichtung 100 automatisch eine Vergiftung eines Detektors 10 zu erkennen vermag. Bevorzugt wird ein Detektor-Kompensator-Paar P.x mit einem Detektor 10.x nicht mehr eingeschaltet, wenn eine erhebliche Vergiftung des Detektors 10.x automatisch erkannt wurde. Weil erfindungsgemäß mehrere Detektor-Kompensator-Paare P.x vorhanden sind, lässt sich die Gasdetektionsvorrichtung 100 trotzdem weiterverwenden. Die Erfindung verlängert also die Lebensdauer der Gasdetektionsvorrichtung 100.

In einer bevorzugten Ausgestaltung wird ein Detektor in Reserve gehalten. Im Ausführungsbeispiel gemäß Figur 5 ist dies der Detektor 10.8, der eine größere wirksame Oberfläche als die übrigen Detektoren 10.1, ..., 10.7 aufweist. "In Reserve halten" bedeutet, dass das Detektor-Kompensator-Paar P.8 mit dem Detektor 10.8 solange nicht eingeschaltet wird, wie kein Verdacht auf eine Vergiftung eines anderen Detektors 10.1, ..., 10.7 detektiert wird.

Möglich ist, dass mindestens ein Detektor-Kompensator-Paar P.1, ..., P.8 vergiftet ist, insbesondere weil Schadstoffe sich auf der erhitzten Oberfläche des Detektors 10.1, ..., 10.8 abgesetzt haben, oder aufgrund einer sonstigen Alterung. In diesem Falle vermag das Paar mit diesem Detektor kein Zielgas zu detektieren, und die Messwerte dieses Paares führen zu einem zu geringen Wert für die Zielgas-Konzentration. In der Regel führen die Messwerte eines vergifteten oder auf andere Weise gealterten Paares aber nicht zu einem deutlich zu großen Wert für die Zielgas-Konzentration.

Die Gasdetektionsvorrichtung 100 des Ausführungsbeispiels detektiert einen Verdacht auf eine Vergiftung bevorzugt wie folgt, und zwar im laufenden Betrieb oder in einer speziellen Überprüfungsphase: Nacheinander schaltet das Steuergerät 9 die Paare P.x ein und wieder aus. Jedes Paar P.x liefert jeweils einen Wert für die Detektionsgröße. Die Auswerteeinheit 6 leitet für jedes eingeschaltete Paar P.x jeweils einen geschätzten Wert con.x für die aktuelle Zielgas-Konfiguration her. Hierfür wendet die Auswerteeinheit 6 einen vorgegebenen funktionalen Zusammenhang auf den Wert der Detektionsgröße an. Die Auswerteeinheit 6 liefert dadurch eine zeitliche Abfolge con.x(1), con.x(2), ... von geschätzten Werten für die aktuelle Zielgas-Konzentration. Jeder Wert con.x(1), con.x(2), ... bezieht sich auf jeweils einen Abtastzeitpunkt t(1), t(2), ... mit t(1) < t(2) < ... Jedes Paar P.x liefert jeweils mindestens einen Wert con.x(i) für die aktuelle Zielgas-Konzentration.

Die Auswerteeinheit 6 prüft, ob die Zielgas-Konzentration zwischen zwei aufeinanderfolgenden Abtastzeitpunkten t(1), t(2), ... stark ansteigt oder abfällt. Zunächst wird die erste Alternative beschrieben, also die Detektion eines starken Anstiegs.

Für die nachfolgende Beschreibung bezeichnet con.a(j) einen Wert für die Zielgas-Konzentration, der aufgrund mindestens eines Detektionsgrößen-Werts des Paars P.a (a = 1 ,..., 7) hergeleitet wurde und sich auf den Abtastzeitpunkt t(j) bezieht (j = 1, 2, ...). Mit con.b(j) wird ein Wert vom Paar P.b bezeichnet (b=1, ..., 7), mit con.8(j) ein Wert vom Paar P.8. Wie bereits erwähnt und in Figur 5 gezeigt, weist der Detektor 10.8 des Paars P.8 eine größere wirksame Oberfläche auf als die übrigen sieben Detektoren 10.1, ..., 10.7. Außerdem wird das Paar P.8 in Reserve gehalten, also nur zur Überprüfung eingeschaltet, und ist daher in geringerem Maße Belastungen durch Schadgase ausgesetzt als die übrigen sieben Detektoren 10.1, ..., 10.7. Daher wird davon ausgegangen, dass das Paar P.8 so lange intakt ist und korrekte Werte liefert, bis die Gasdetektionsvorrichtung 100 regulär überprüft wird.

Die Auswerteeinheit 6 prüft, ob eine Differenz con.b(i) - con.a(i-1) der Zielgas-Konzentrations-Werte an den beiden aufeinander folgenden Abtastzeitpunkten t(i-1) und t(i) größer als eine vorgegebene absolute oder relative Schranke ist, wobei die beiden Werte con.a(i-1) und con.b(i) aufgrund von Messungen von zwei verschiedenen Paaren P.a und P.b ermittelt wurden und wobei diese Schranke vom Abstand zwischen t(i-1) und t(i) abhängen kann. Das Paar P.a liefert den Wert con.a(i-1), das Paar P.b den Wert con.b(i).

Ein gemessener starker Anstieg der Zielgas-Konzentration zwischen den beiden Abtastzeitpunkten t(i-1) und t(i) kann folgende Ursachen haben:
- Die Zielgas-Konzentration ist tatsächlich zwischen den beiden Abtastzeitpunkten t(i-1) und t(i) stark angestiegen, beispielsweise weil in der Zeitspanne zwischen den beiden Abtastzeitpunkten t(i-1) und t(i) eine relevante Menge von Zielgas ausgetreten ist.
- In Wirklichkeit lag bereits zum früheren Abtastzeitpunkt eine hohe Zielgas-Konzentration vor. Jedoch ist das Paar P.a so stark vergiftet, dass diese hohe Zielgas-Konzentration nur aufgrund einer Messung des Paars P.a nicht mehr detektiert werden kann.

Diese beiden Situationen werden nunmehr automatisch voneinander unterschieden. Um die Gasdetektionsvorrichtung 100 zu überprüfen, schaltet das Steuergerät 9 das Paar P.8 an.

Die Auswerteeinheit 6 leitet aus Messwerten des Paars P.8 einen Wert con.8(i+1) für die Zielgas-Konzentration her. Der Wert con.8(i+1) bezieht sich auf einen nachfolgenden Abtastzeitpunkt t(i+1) und wird als korrekt angesehen. Die Auswerteeinheit 6 vergleicht diesen Wert con.8(i+1) mit den beiden Werten con.a(i-1) und con.b(i). Abhängig vom Ergebnis der Auswerteeinheit 6 löst das Steuergerät 9 folgende Schritte aus:
- Falls der größere Wert con.b(i) ausreichend genau mit dem Wert con.8(i+1) übereinstimmt, so lag zumindest zu den Abtastzeitpunkten t(i) und t(i+1) tatsächlich eine höhere Zielgas-Konzentration con.b(i) bzw. con.8(i+1) vor als zum Abtastzeitpunkt t(i-1), und das Paar P.b funktioniert noch.
- Zu prüfen ist, ob der niedrige Wert con.a(i-1) am früheren Abtastzeitpunkt t(i-1) korrekt ist oder nicht. Das Steuergerät 9 schaltet das Paar P.a an, und aufgrund der Messung des Paars P.a liefert die Auswerteeinheit 6 einen Wert con.a(t+2), der sich auf den Abtastzeitpunkt t(i+2) bezieht. Liefert auch das Paar P.a einen großen Wert con.a(t+2), so ist tatsächlich die Zielgas-Konzentration zwischen den beiden Abtastzeitpunkten t(i-1) und t(i) stark angestiegen, und auch das Paar P.a funktioniert.

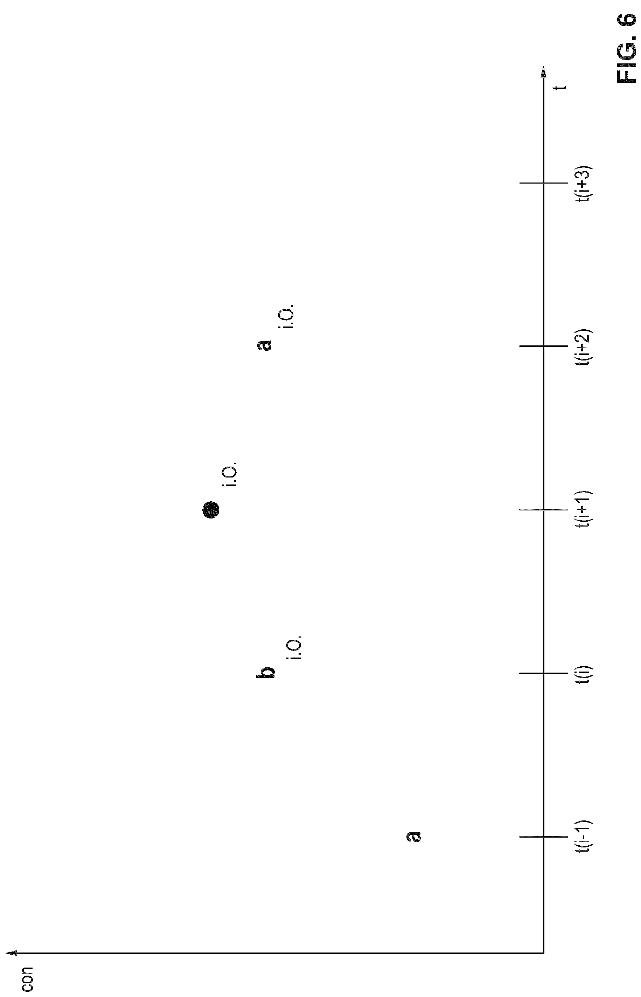

Figur 6 veranschaulicht diese Abfolge. Auf der x-Achse ist die Zeit t aufgetragen, auf der y-Achse die jeweils gemessene Zielgas-Konzentration con. Messwerte aufgrund einer Messung des Paars P.a werden mit einem a bezeichnet, Messwerte aufgrund einer Messung des Paars P.b mit einem b und Messwerte aufgrund einer Messung des Paars P.8 mit einem Vollkreis.

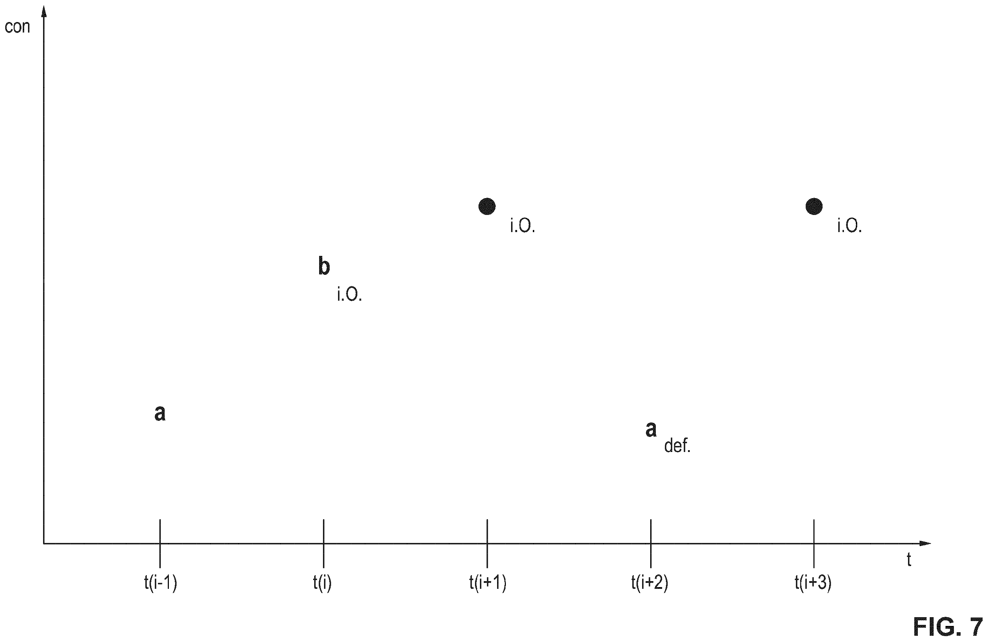

Liefert das Paar P.a hingegen einen kleinen Wert con.a(i+2), so schaltet das Steuergerät erneut das Paar P.8 an. Dadurch wird ein Wert con.8(i+3) für einen Abtastzeitpunkt t(t+3) geliefert. Liefert das Paar P.8 erneut einen großen Wert con.8(i+3), so ist der Wert con.a(i+2) falsch, und das Paar P.a ist defekt und wird nicht weiter benutzt. Figur 7 zeigt diese Situation.

Liefert das Paar P.8 hingegen ebenfalls einen kleinen Wert con.8(i+3), so ist die Zielgas-Konzentration nach dem Abtastzeitpunkt t(i+2) wieder stark abgefallen. Eine mögliche Ursache hierfür ist, dass die Gasdetektionsvorrichtung 100 aus einem Bereich mit einer hohen Zielgas-Konzentration in einen Bereich mit einer niedrigen Zielgas-Konzentration oder ohne Zielgas bewegt worden ist. Auch das Paar P.a wird dann weiterbenutzt.

Nunmehr wird die zweite Alternative beschrieben, also die Detektion eines starken Abfalls. Genauer gesagt: Der Wert con.b(i) für den Abtastzeitpunkt t(i) ist deutlich geringer als der Wert con.a(i-1) für den Abtastzeitpunkt t(i-1). Die Auswerteeinheit 6 hat den Wert con.b(i) aufgrund von Messwerten des Paars P.b hergeleitet, den Wert con.a(i-1) aufgrund von Messwerten des Paars P.a. Wiederum wird zur Überprüfung ein Wert con.8(i+1) verwendet, der sich auf den Abtastzeitpunkt t(i+1) bezieht.

Zunächst wird die Situation beschrieben, dass der als korrekt angesehene Wert con.8(i+1) etwa so groß wie oder sogar noch größer ist als der größere Wert con.a(t-1). In diesem Fall schaltet das Steuergerät 9 erneut das Paar P.b ein. Der Messwert beim erneuten Einschalten führt zu einem Wert con.b(i+2), der sich auf den Abtastzeitpunkt t(i+2) bezieht.

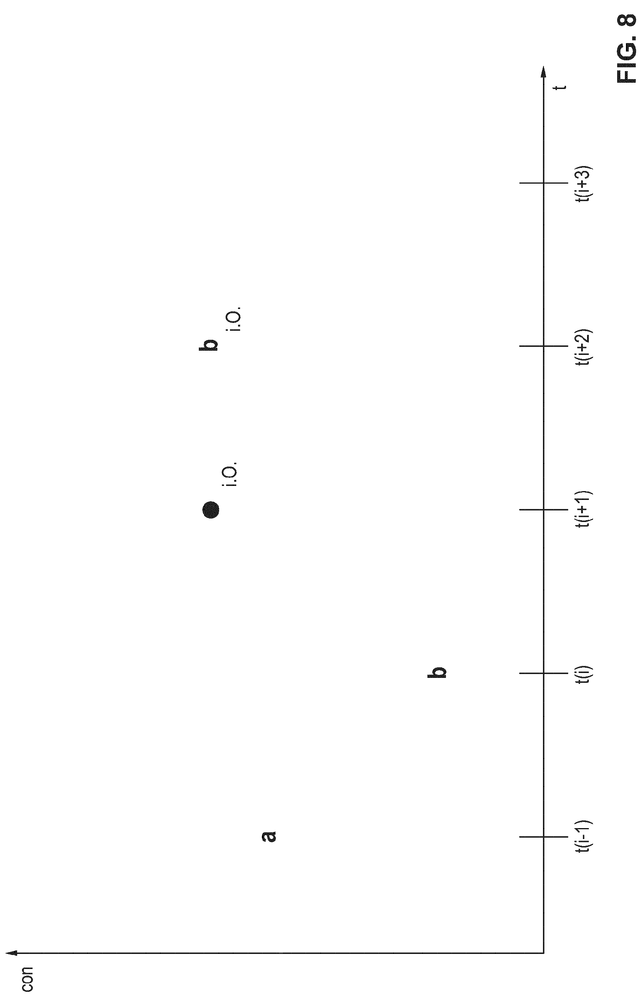

Falls der Wert con.b(i+2) für den Abtastzeitpunkt t(i+2) etwa so groß ist wie der als korrekt angesehene Wert con.8(i+1), so wird das Paar P.b als intakt angesehen und weiter verwendet. Diese Situation veranschaulicht Figur 8.

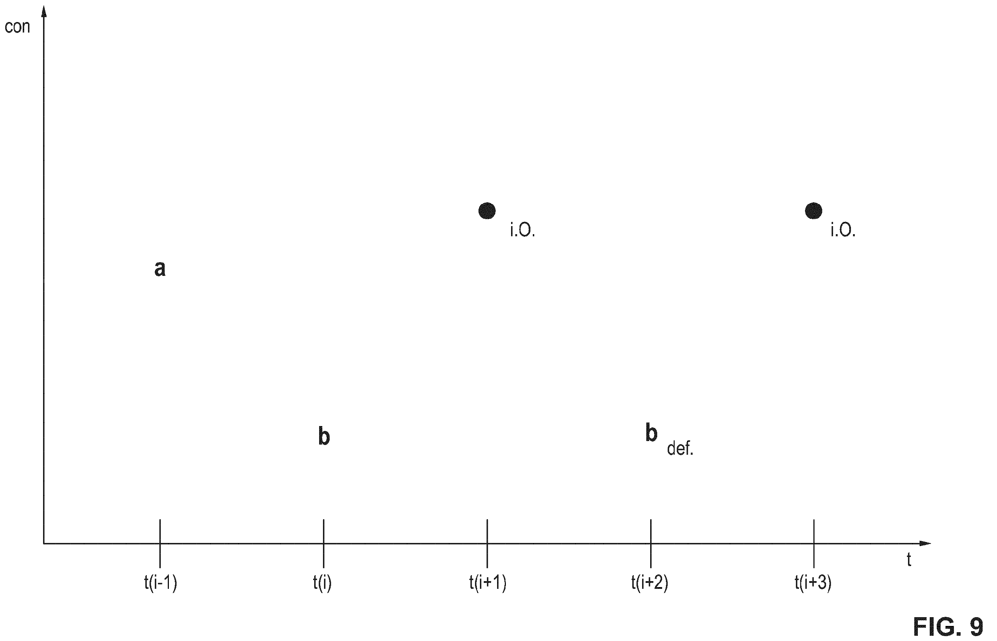

Falls hingegen auch der Wert con.b(i+2) für den Abtastzeitpunkt t(i+2) deutlich geringer ist, beispielsweise etwa genauso groß wie der Wert con.b(i) für den Abtastzeitpunkt t(i), wird bevorzugt erneut das Paar P.8 eingeschaltet. Dadurch wird ein Wert con.8(i+3) für einen Abtastzeitpunkt t+3 geliefert. Falls dieser Wert con.8(i+3) wiederum groß ist, so ist das Paar P.b defekt und wird nicht weiter benutzt. Diese Situation zeigt Figur 9.

Wie bereits dargelegt, wird im Ausführungsbeispiel das Paar P.8 nur zur Überprüfung eingeschaltet. In einer Ausgestaltung werden alle übrigen Paare P.1, ..., P.7 gleich oft und jeweils gleich lange eingeschaltet, beispielsweise reihum.

In einer anderen Ausgestaltung wird wie folgt vorgegangen:
Zunächst wird ausschließlich das Paar P.1 verwendet, wobei das Paar P.1 N-mal eingeschaltet und wieder ausgeschaltet wird., N >= 2 Die übrigen Paare P.2, ..., P.8 bleiben ausgeschaltet. Anschließend wird das Paar P.2 einmal eingeschaltet und wieder ausgeschaltet. Die letzten beiden Messungen con.1(i-1) und con.2(i) werden miteinander verglichen, beispielsweise so, wie dies weiter oben mit Bezug auf Figur 6 bis Figur 9 beschrieben wurde. P.1 fungiert als das Paar P.a, P.2 als das Paar P.b. Bei einer großen Abweichung wird das Paar P.8 mindestens einmal eingeschaltet und wieder ausgeschaltet.

Falls die Überprüfung ergibt, dass beide Paare P.1 und P.2 noch intakt sind, so wird die gerade beschriebene Abfolge wiederholt, also erneut zunächst das Paar P.1 N-mal eingeschaltet und wieder ausgeschaltet und anschließend einmal das Paar P.2. Die übrigen Paare bleiben ausgeschaltet. Das Paar P.1 wird also N-mal stärker belastet als das Paar P.2.

Weil das Paar P.1 N-mal stärker belastet wird, vergiftet das Paar P.1 in der Regel rascher als die anderen Paare P.2 bis P.8. Falls detektiert wird, dass das Paar P.1 vergiftet ist, so wird es nicht weiter verwendet. Die gerade beschriebene Abfolge wird nunmehr mit den Paaren P.2 und P.3 durchgeführt. Zunächst wird also wieder eine Abfolge durchgeführt, bei der das Paar P.2 N-mal eingeschaltet und wieder ausgeschaltet wird und anschließend einmal das Paar P.3. Die beiden letzten Werte für die Zielgas-Konzentration werden wieder miteinander verglichen. Das Paar P.2 fungiert als das Paar P.a, das Paar P.3 als das Paar P.b.

Dieses Vorgehen wird durchgeführt, bis die Paare P.1 bis P.6 vergiftet sind, so dass eine Selbstkontrolle nicht mehr möglich ist. Möglich ist auch, das eine Paar N-mal und das andere Paar M-mal einzuschalten, wobei N > M > 1 gilt. Das oben Gesagte gilt entsprechend.

### Bezugszeichenliste

| | |
|---|---|
| 1 | stabiles und hitzebeständiges inneres Gehäuse, nimmt den Detektor 10.p und den Kompensator 11.p, 11.m auf |
| 2 | Flammschutz vor der Öffnung Ö |
| 3 | elektrische Leitung, verbindet die Spannungsversorgungseinheit 42 mit dem Detektor 10.p und dem Kompensator 11.p, 11.m |
| 4 | äußeres Gehäuse aus einem stabilen Kunststoff, umgibt die übrigen Bestandteile der Gasdetektionsvorrichtung 100 |
| 6 | signalverarbeitendes Steuergerät, umfasst die Auswerteeinheit 9 |
| 7.10 | ansteuerbarer Schalter, bewirkt, dass am Detektor 10.p eine gepulste elektrische Spannung U10 anliegt |
| 7.11 | ansteuerbarer Schalter, bewirkt, dass am Kompensator 11.m eine gepulste elektrische Spannung U11 anliegt |
| 8.10 | Spannungs-Aktor für den Detektor 10.p |
| 8.11 | Spannungs-Aktor für den Kompensator 11.m |
| 9 | Auswerteeinheit, leitet die Zielgas-Konzentration her |
| 10.1, 10.2, ... | Detektoren |
| 10.8 | Detektor mit einer größeren wirksamen Oberfläche |
| 10.m | flacher Detektor |
| 10.p | Detektor, der als kugelförmiger Pellistor ausgestaltet ist |
| 11 | Kompensator |
| 11.m | flacher Kompensator |
| 11.p | Kompensator, der als kugelförmiger Pellistor ausgestaltet ist |
| 15.1, .... | Aussparungen in der Trägerplatte 31 und der Schutzschicht 35 |
| 17 | optionale thermische Barriere zwischen dem Detektor 10.p und dem Kompensator 11.m |
| 20 | heizendes Segment des Detektors 10.p |
| 21 | Keramikummantelung um das heizende Segment 20 |
| 22 | Platte, welche die elektrischen Kontaktierungen 36 trägt |
| 23 | katalytische Beschichtung der Keramikummantelung 21 des Detektors 10.p |
| 24 | katalytische Beschichtung des Detektors 10.m auf der Schutzschicht 35 |
| 30 | elektrisch leitendes Bauteil in Form einer Leiterbahn mit dem heizenden Segment 32 und der elektrischen Kontaktierung 46, einmal im Detektor 10.m und einmal im Kompensator 11.m vorhanden |
| 31 | Trägerplatte in Form einer Platine, stellt die elektrischen Kontaktierungen für die Detektoren 10.m, 10.1, ..., 10.8 und den Kompensator 11.m, 11 bereit, von der Schutzschicht 35 bedeckt, weist die Aussparungen 15.1, ... auf |
| 32 | heizendes Segment des Bauteils 30 |
| 35 | Schutzschicht für die Leiterbahn 30, weist die Aussparungen 15.1, ... auf |
| 36 | elektrische Kontaktierungen des heizenden Segments 20 |
| 40 | Spannungs-Sensor, misst die Brückenspannung ΔU = (U10 - U11) / 2 |
| 41 | Stromstärken-Sensor, misst die Stromstärke I3 |
| 41.10 | Stromstärken-Sensor, misst die Stromstärke I10 |
| 41.11 | Stromstärken-Sensor, misst die Stromstärke I11 |
| 42 | Spannungsversorgungseinheit |
| 46 | elektrische Kontaktierung des Bauteils 30 |
| 100 | Gasdetektionsvorrichtung, umfasst die Detektions-Anordnung DA, das Steuergerät 9 mit der Auswerteeinheit 6, die Spannungsversorgungseinheit 42 und die beiden Gehäuse 1 und 4 mit der Öffnung 2 |
| con.a(j) | Wert für die Zielgas-Konzentration, die aufgrund eines Messwerts vom Paar P.a hergeleitet wird und sich auf den Abtastzeitpunkt t(j) bezieht (a=1,...,7;j=1,2,...) |
| con.b(j) | Wert für die Zielgas-Konzentration, die aufgrund eines Messwerts vom Paar P.b hergeleitet wird und sich auf den Abtastzeitpunkt t(j) bezieht (b=1,...,7;j=1,2,...) |
| con.8(j) | Wert für die Zielgas-Konzentration, die aufgrund eines Messwerts vom Paar P.8 hergeleitet wird und sich auf den Abtastzeitpunkt t(j) bezieht (j=1,2,...) |
| DA | Detektions-Anordnung, umfasst die acht Detektoren 10.1, ..., 10.8, den Kompensator 11, die Platine 31 und bevorzugt die elektrischen Kontaktierungen |
| I3 | Stromstärke des Stroms durch die Leitung 3, den Detektor 10.p und den Kompensator 11.p, wird vom Stromstärken-Sensor 41 gemessen |
| I10 | Stromstärke des Stroms durch den Detektor 10.p, wird vom Stromstärken-Sensor 41.10 gemessen |
| I11 | Stromstärke des Stroms durch den Kompensator 11.m, wird vom Stromstärken-Sensor 41.11 gemessen |
| Ö | Öffnung im äußeren Gehäuse 4 und im inneren Gehäuse 1, durch den Flammschutz 2 gesichert |
| P.1, P.2, ... | Detektor-Kompensator-Paar, umfasst jeweils einen Detektor 10.1, ..., 10.8 und den Kompensator 11 |
| R10 | elektrischer Widerstand des Detektors 10.p |
| R11 | elektrischer Widerstand des Kompensators 11.p |
| R20 | elektrischer Widerstand, parallel zum Detektor 10.p geschaltet |
| R21 | elektrischer Widerstand, parallel zum Kompensator 11.p geschaltet |
| t(1), t(2), ... | Abtastzeitpunkte |
| U10 | elektrische Spannung, die am Detektor 10.p anliegt, bevorzugt eine gepulste Spannung |
| U11 | elektrische Spannung, die am Kompensator 11.p, 11.m anliegt, bevorzugt eine gepulste Spannung |
| U42 | elektrische Spannung, die an der Spannungsversorgungseinheit 42 anliegt |
| ΔU | Brückenspannung, vom Spannungs-Sensor 40 gemessen, ist gleich (U10 - U11) / 2 |

## Patentansprüche

1. Gasdetektionsvorrichtung (100), die dazu ausgestaltet ist, in einer Gasprobe das Vorhandensein mindestens eines brennbaren Zielgases (CH₄) zu detektieren und / oder die Konzentration des Zielgases (CH₄) in der Gasprobe zu ermitteln,
wobei die Gasdetektionsvorrichtung (100)
- eine Detektions-Anordnung (DA) mit mindestens drei Detektionseinheiten (10.m, 10.p, 10.1, ..., 10.8, 11.m, 11.p, 11),
- eine Messeinheit (40, 40.10, 40.11, 41, 41.10, 41.11) und
- eine signalverarbeitende Auswerteeinheit (6)
umfasst,
wobei mindestens zwei verschiedene Detektionseinheiten Detektoren (10.m, 10.p, 10.1, ..., 10.8) sind und mindestens eine weitere Detektionseinheit ein Kompensator (11.m, 11.p, 11) ist,
so dass mindestens zwei verschiedene Detektor-Kompensator-Paare (P.1, ..., P.8) mit jeweils genau einem Detektor (10.m, 10.p, 10.1, ..., 10.8) und genau einem Kompensator (11.m, 11.p, 11) gebildet werden,
wobei die Anzahl der Detektoren größer als die Anzahl der Kompensatoren ist,
wobei der oder mindestens ein Kompensator (11) zu mindestens zwei verschiedenen Detektor-Kompensator-Paaren (P.1, ..., P.8) gehört,
**dadurch gekennzeichnet, dass** die Gasdetektionsvorrichtung (100) dazu ausgestaltet ist, jedes Detektor-Kompensator-Paar (P.1, ..., P.8) unabhängig von jedem anderen Detektor-Kompensator-Paar einzuschalten und auszuschalten,
wobei die Gasdetektionsvorrichtung (100) so ausgestaltet ist, dass zu jedem Zeitpunkt höchstens ein Detektor-Kompensator-Paar (P.1, ..., P.8) eingeschaltet und das oder jedes andere Detektor-Kompensator-Paar ausgeschaltet ist,
wobei bei eingeschaltetem Detektor-Kompensator-Paar (P.1, ..., P.8) wenigstens zeitweise eine elektrische Spannung (U10) dauerhaft oder gepulst am Detektor (10.m, 10.p, 10.1, ..., 10.8) und wenigstens zeitweise eine elektrische Spannung (U11) dauerhaft oder gepulst am Kompensator (11.m, 11.p, 11) anliegt,
wobei die jeweils anliegenden elektrischen Spannungen (U10, U11) eine Erhitzung des Detektors (10.m, 10.p, 10.1, ..., 10.8) und eine Erhitzung des Kompensators (11.m, 11.p, 11) bewirken,
wobei jeder Detektor (10.m, 10.p, 10.1, ..., 10.8) so ausgestaltet ist, dass eine Erhitzung des Detektors (10.m, 10.p, 10.1, ..., 10.8) eine Oxidierung von Zielgas (CH₄) bewirkt, wobei das Oxidieren Wärmeenergie freisetzt, welche auf den Detektor (10.m, 10.p, 10.1, ..., 10.8) einwirkt,
wobei der oder jeder Kompensator (11.m, 11.p, 11) so ausgestaltet ist, dass eine Erhitzung des oder jedes Kompensators (11.m, 11.p, 11) eine geringere Oxidierung von Zielgas (CH₄) als die Erhitzung eines Detektors (10.m, 10.p, 10.1, ..., 10.8) oder sogar gar keine Oxidierung bewirkt,
wobei jedes Detektor-Kompensator-Paar (P.1, ..., P.8) eine Detektionsgröße (ΔU, U10-U11) aufweist, die mit der Wärmeenergie korreliert, die beim Oxidieren durch das Detektor-Kompensator-Paar (P.1, ..., P.8) freigesetzt wird,
wobei die Messeinheit (40, 40.10, 40.11, 41, 41.10, 41.11) dazu ausgestaltet ist, für jedes Detektor-Kompensator-Paar (P.1, ..., P.8) zu messen, welchen Wert die Detektionsgröße (ΔU, U10-U11) für dieses Detektor-Kompensator-Paar (P.1, ..., P.8) annimmt,
wobei die Auswerteeinheit (6) dazu ausgestaltet ist, automatisch
- für jedes eingeschaltete Detektor-Kompensator-Paar (P.1, ..., P.8) abhängig von dem gemessenen Wert, den die Detektionsgröße (ΔU, U10-U11) für das Detektor-Kompensator-Paar (P.1, ..., P.8) annimmt,
über das Vorhandensein des Zielgases (CH₄) zu entscheiden und / oder die Zielgas-Konzentration zu ermitteln, und
wobei die Gasdetektionsvorrichtung (100) dazu ausgestaltet ist,
- jedes Detektor-Kompensator-Paar (P.1, ..., P.8) einzuschalten,
- für das eingeschaltete Detektor-Kompensator-Paar (P.1, ..., P.8) eine Messung der jeweiligen Detektionsgröße (ΔU, U10-U11) und eine Auswertung zu bewirken und
- das Detektor-Kompensator-Paar (P.1, ..., P.8) wieder auszuschalten.

2. Gasdetektionsvorrichtung (100) nach einem der vorhergehenden Ansprüche,
**dadurch gekennzeichnet, dass**
in einem Einsatz-Zeitraum der Gasdetektionsvorrichtung (100) mindestens ein erstes Detektor-Kompensator-Paar (P.1, ..., P.7) insgesamt für eine längere Zeitdauer eingeschaltet ist als ein zweites Detektor-Kompensator-Paar (P.8).

3. Gasdetektionsvorrichtung (100) nach Anspruch 2,
**dadurch gekennzeichnet, dass**
die Auswerteeinheit (6) dazu ausgestaltet ist,
die Zielgas-Konzentration zu ermitteln,
den Zielgas-Konzentrations-Schätzwert, den das erste Detektor-Kompensator-Paar (P.1, ..., P.7) liefert, mit dem Zielgas-Konzentrations-Schätzwert, den das zweite Detektor-Kompensator-Paar (P.8) liefert, zu vergleichen, und
dann, wenn die beiden Schätzwerte sich um mehr als eine vorgegebene Schranke voneinander unterscheiden, einen der Schritte auszulösen,
- den Wert eines Parameters zu verändern, wobei die Auswerteeinheit (6) diesen Parameter-Wert verwendet, um abhängig von den gemessenen Werten des ersten Detektor-Kompensator-Paars (P.1, ..., P.7) über das Vorhandensein von Zielgas (CH₄) zu entscheiden und / oder die Zielgas-Konzentration zu ermitteln, oder
- das erste Detektor-Kompensator-Paar (P.1, ..., P.7) zu deaktivieren.

4. Gasdetektionsvorrichtung (100) nach Anspruch 2 oder Anspruch 3,
**dadurch gekennzeichnet, dass**
die Gasdetektionsvorrichtung (100) dazu ausgestaltet ist,
im Einsatz-Zeitraum das erste Detektor-Kompensator-Paar (P.1, ..., P.7) N1 mal und das zweite Detektor-Kompensator-Paar (P.8) N2 mal einzuschalten und wieder auszuschalten,
wobei N1 und N2 zwei Anzahlen sind und N1 größer als N2 ist.

5. Gasdetektionsvorrichtung (100) nach einem der Ansprüche 2 bis 4,
**dadurch gekennzeichnet, dass**
die Gasdetektionsvorrichtung (100) dazu ausgestaltet ist, im Einsatz-Zeitraum mindestens einmal eine Abfolge durchzuführen,
wobei während der Abfolge
- zunächst das erste Detektor-Kompensator-Paar (P.1, ..., P.7) N-mal eingeschaltet und wieder ausgeschaltet wird und
- dann das zweite Detektor-Kompensator-Paar (P.8) M-mal eingeschaltet und wieder ausgeschaltet wird,
wobei M und N zwei Anzahlen sind und 0 < M < N gilt.

6. Gasdetektionsvorrichtung (100) nach einem der vorhergehenden Ansprüche,
**dadurch gekennzeichnet, dass**
mindestens ein Detektor-Kompensator-Paar (P.1, ..., P.8) so ausgestaltet ist, dass beide Detektionseinheiten (10, 11) des Detektor-Kompensator-Paars (P.1, ..., P.8) jeweils eine Detektionsgröße (U10, U11) aufweisen,
wobei die jeweilige Detektionsgröße (U10, U11) mit einer Temperatur der Detektionseinheit (10, 11) korreliert und
wobei die Messeinheit dazu ausgestaltet ist, bei eingeschaltetem Detektor-Kompensator-Paar (P.1, ..., P.8) zu messen, welchen Wert die Detektionsgröße (U10) für den Detektor (10.1, ..., 10.8) und welchen Wert die Detektionsgröße (U11) für den Kompensator (11) annimmt.

7. Gasdetektionsvorrichtung (100) nach Anspruch 6,
**dadurch gekennzeichnet, dass**
die Gasdetektionsvorrichtung (100) dazu ausgestaltet ist, das Detektor-Kompensator-Paar (P.1, ..., P.8) mit den beiden Detektionsgrößen (U10, U11) dergestalt zu betreiben,
dass zunächst die eine Detektionseinheit (10.1, ..., 10.8) und anschließend die andere Detektionseinheit (11) des Detektor-Kompensator-Paars (P.1, ..., P.8) eingeschaltet und wieder ausgeschaltet wird,
so dass zu jedem Zeitpunkt in einer Aktivierungs-Zeitspanne, in der das Detektor-Kompensator-Paar (P.1, ..., P.8) eingeschaltet ist,
höchstens eine Detektionseinheit (10.1, ..., 10.8, 11) des Detektor-Kompensator-Paars (P.1, ..., P.8) eingeschaltet ist.

8. Gasdetektionsvorrichtung (100) nach Anspruch 6 oder Anspruch 7,
**dadurch gekennzeichnet, dass**
die Gasdetektionsvorrichtung (100) dazu ausgestaltet ist, das Detektor-Kompensator-Paar (P.1, ..., P.8) mit den beiden Detektionsgrößen (U10, U11) dergestalt zu betreiben,
dass die Messeinheit (40, 40.10, 40.11, 41, 41.10, 41.11) in einer Aktivierungs-Zeitspanne, in der das Detektor-Kompensator-Paar (P.1, ..., P.8) eingeschaltet ist,
den Wert des der Detektionsgröße (U10) des Detektors (10.1, ..., 10.8) häufiger misst als den Wert der Detektionsgröße (U11) des Kompensators (11),
bevorzugt den Wert der Detektionsgröße (U11) des Kompensators (11) genau einmal misst.

9. Gasdetektionsvorrichtung (100) nach einem der vorhergehenden Ansprüche,
**dadurch gekennzeichnet, dass**
die Detektions-Anordnung (DA) sich in einer Ebene erstreckt und die maximale Abmessung senkrecht zu dieser Ebene höchstens 20%, bevorzugt höchstens 10%, besonders bevorzugt höchstens 5%, der maximalen Abmessung in der Ebene beträgt,
wobei gesehen in eine Betrachtungsrichtung senkrecht auf der Ebene die Detektionseinheiten (10.m, 10.p, 10.1, ..., 10.8, 11.m, 11.p, 11) überlappungsfrei in mindestens einer Reihe angeordnet sind.

10. Gasdetektionsvorrichtung (100) nach Anspruch 9,
**dadurch gekennzeichnet, dass**
in der oder einer Reihe, in welche mehrere Detektionseinheiten (10.m, 10.p, 10.4, 10.5, 10.2, 10.7, 11.m, 11.p, 11) angeordnet sind, der oder ein Kompensator (11) zwischen zwei Detektoren (10.4, 10.5, 10.2, 10.7) angeordnet ist.

11. Gasdetektionsvorrichtung (100) nach einem der vorhergehenden Ansprüche, **dadurch gekennzeichnet, dass**
die Detektions-Anordnung (DA) mindestens drei Detektoren (10.1, ..., 10.8) umfasst,
wobei die mindestens drei Detektoren (10.1, ..., 10.8) und der oder ein Kompensator (11) sich in derselben Ebene erstrecken und
in dieser Ebene der oder ein Kompensator (11) zwischen mindestens zwei (10.4, 10.5, 10.2, 10.7) der mindestens drei Detektoren (10.1, ..., 10.8) angeordnet ist

12. Gasdetektionsvorrichtung (100) nach einem der vorhergehenden Ansprüche,
**dadurch gekennzeichnet, dass**
die Detektions-Anordnung eine Platine (31) umfasst und
jede Detektionseinheit (10.m, 11.m) jeweils ein Heizelement (32) umfasst,
wobei das Heizelement (32) einer Detektionseinheit (10.m, 11.m) sich erhitzt, wenn eine elektrische Spannung (U10, U11) an der Detektionseinheit (10.m, 11.m) anliegt,
wobei die Heizelemente (32) auf und / oder in der Platine (31) angeordnet sind,
wobei die Platine (31) für jedes Heizelement (32) jeweils eine elektrische Kontaktierung (46) bereitstellt und
wobei das jeweilige Heizelement (32) jedes Detektors (10.m) mit jeweils einer Beschichtung (35) bedeckt ist, welche ein katalytisches Material aufweist oder auf welches ein katalytisches Material (24) aufgebracht ist.

13. Gasdetektionsvorrichtung (100) nach einem der vorhergehenden Ansprüche,
**dadurch gekennzeichnet, dass**
die Detektions-Anordnung (DA) ein drittes Detektor-Kompensator-Paar (P.8) umfasst und die Auswerteeinheit (6) dazu ausgestaltet ist, die Zielgas-Konzentration zu ermitteln,
wobei die Auswerteeinheit (6) dazu ausgestaltet ist,
- den Zielgas-Konzentrations-Schätzwert, den das erste Detektor-Kompensator-Paar (P.1) liefert, mit dem Zielgas-Konzentrations-Schätzwert, den das zweite Detektor-Kompensator-Paar (P.2, ..., P.7) liefert, zu vergleichen, und
- das dritte Detektor-Kompensator-Paar (P.8) nur dann wenigstens zeitweise einzuschalten, wenn die beiden Schätzwerte sich um mehr als eine vorgegebene Schranke voneinander unterscheiden.

14. Gasdetektionsvorrichtung (100) nach Anspruch 13,
**dadurch gekennzeichnet, dass**
jeder Detektor (10.m, 10.p, 10.1, ..., 10.8) eine wirksame Oberfläche, die in Kontakt mit einer Gasprobe kommt, aufweist,
wobei der Detektor (10.8) des dritten Detektor-Kompensator-Paars (P.8) eine größere wirksame Oberfläche aufweist als der Detektor des ersten Detektor-Kompensator-Paars (P.1) und als der Detektor des zweiten Detektor-Kompensator-Paars (P.2, ..., P.8).

15. Gasdetektionsvorrichtung (100) nach einem der vorhergehenden Ansprüche, **dadurch gekennzeichnet, dass**
für jedes Detektor-Kompensator-Paar (P.1, ..., P.8) jeweils ein Nullwert vorgegeben ist, das ist ein Wert, den die Detektionsgröße (ΔU, U10-U11) für dieses Detektor-Kompensator-Paar (P.1, ..., P.8) bei Abwesenheit des Zielgases (CH₄) annimmt,
wobei die Auswerteeinheit (6) dazu ausgestaltet ist, für jedes Detektor-Kompensator-Paar (P.1, ..., P.8) die Differenz zwischen dem Wert, den die Detektionsgröße (ΔU, U10-U11) annimmt, und dem vorgegebenen Nullwert der Detektionsgröße zu berechnen, und
für jedes eingeschaltete Detektor-Kompensator-Paar (P.1, ..., P.8) abhängig von der Differenz zwischen den gemessenen Detektionsgrößen-Wert und dem Nullwert
über das Vorhandensein des Zielgases (CH₄) zu entscheiden und / oder die Zielgas-Konzentration zu ermitteln.

16. Gasdetektionsverfahren zum Detektieren des Vorhandenseins mindestens eines brennbaren Zielgases (CH₄) in einer Gasprobe und / oder zum Ermitteln der Konzentration des Zielgases (CH₄) in der Gasprobe unter Verwendung einer Gasdetektionsvorrichtung (100), die
- eine Detektions-Anordnung (DA) mit mindestens drei Detektionseinheiten (10.m, 10.p, 10.1, ..., 10.8, 11.m, 11.p, 11) und
- eine Messeinheit (40, 40.10, 40.11, 41, 41.10, 41.11) umfasst,
wobei mindestens zwei verschiedene Detektionseinheiten Detektoren (10.m, 10.p, 10.1, ..., 10.8) sind und mindestens eine weitere Detektionseinheit ein Kompensator (11.m, 11.p, 11) ist,
so dass mindestens zwei verschiedene Detektor-Kompensator-Paare (P.1, ..., P.8) mit jeweils genau einem Detektor (10.m, 10.p, 10.1, ..., 10.8) und genau einem Kompensator (11.m, 11.p, 11) gebildet werden,
wobei die Anzahl der Detektoren größer als die Anzahl der Kompensatoren ist,
wobei der oder mindestens ein Kompensator (11) zu mindestens zwei verschiedenen Detektor-Kompensator-Paaren (P.1, ..., P.8) gehört,
**dadurch gekennzeichnet, dass** das Verfahren die automatisch durchgeführten Schritte umfasst, dass
- mindestens ein, bevorzugt jedes, Detektor-Kompensator-Paar (P.1, ..., P.8) eingeschaltet wird,
- für das eingeschaltete Detektor-Kompensator-Paar (P.1, ..., P.8) eine Messung der Detektionsgröße (ΔU, U10-U11) und eine Auswertung bewirkt wird und
- das Detektor-Kompensator-Paar (P.1, ..., P.8) wieder ausgeschaltet wird,
wobei zu jedem Zeitpunkt höchstens ein Detektor-Kompensator-Paar (P.1, ..., P.8) eingeschaltet und das oder jedes andere Detektor-Kompensator-Paar ausgeschaltet ist,
wobei bei eingeschaltetem Detektor-Kompensator-Paar (P.1, ..., P.8) wenigstens zeitweise eine elektrische Spannung (U10) am Detektor (10.m, 10.p, 10.1, ..., 10.8) und wenigstens zeitweise eine elektrische Spannung (U11) am Kompensator (11.m, 11.p, 11) anliegt,
wobei die jeweils anliegenden elektrischen Spannungen (U10, U11) eine Erhitzung des Detektors (10.m, 10.p, 10.1, ..., 10.8) und eine Erhitzung des Kompensators (11.m, 11.p, 11) bewirken,
wobei eine Erhitzung des Detektors (10.m, 10.p, 10.1, ..., 10.8) eine Oxidierung von Zielgas (CH₄) bewirkt, wobei das Oxidieren Wärmeenergie freisetzt, welche auf den Detektor (10.m, 10.p, 10.1, ..., 10.8) einwirkt,
wobei eine Erhitzung des Kompensators (11.m, 11.p, 11) eine geringere Oxidierung als die Erhitzung eines Detektors (10.m, 10.p, 10.1, ..., 10.8) oder sogar gar keine Oxidierung bewirkt,
wobei jedes Detektor-Kompensator-Paar (P.1, ..., P.8) eine Detektionsgröße (ΔU, U10-U11) aufweist, die mit der Wärmeenergie korreliert, die beim Oxidieren durch das Detektor-Kompensator-Paar (P.1, ..., P.8) freigesetzt wird, und
wobei das Verfahren die weiteren automatisch durchgeführten Schritte umfasst, dass für jedes eingeschaltete Detektor-Kompensator-Paar (P.1, ..., P.8)
- die Messeinheit (40, 40.10, 40.11, 41, 41.10, 41.11) misst, welchen Wert die Detektionsgröße (ΔU, U10-U11) für dieses Detektor-Kompensator-Paar (P.1, ..., P.8) annimmt, und
- abhängig von dem gemessenen Wert, den die Detektionsgröße (ΔU, U10-U11) für das eingeschaltete Detektor-Kompensator-Paar (P.1, ..., P.8) annimmt,
über das Vorhandensein des Zielgases (CH₄) entschieden und / oder die Zielgas-Konzentration ermittelt wird.

## Claims

1. Gas detection device (100) which is configured to detect the presence of at least one combustible target gas (CH₄) in a gas sample and/or to determine the concentration of the target gas (CH₄) in the gas sample,
wherein the gas detection device (100) comprises
- a detection arrangement (DA) having at least three detection units (10.m, 10.p, 10.1, ..., 10.8, 11.m, 11.p, 11),
- a measuring unit (40, 40.10, 40.11, 41, 41.10, 41.11), and
- a signal-processing evaluation unit (6),
wherein at least two different detection units are detectors (10.m, 10.p, 10.1, ..., 10.8) and at least one additional detection unit is a compensator (11.m, 11.p, 11),
so that at least two different detector-compensator pairs (P.1, ..., P.8) are formed having exactly one detector (10.m, 10.p, 10.1, ..., 10.8) and exactly one compensator (11.m, 11.p, 11) in each case,
wherein the number of detectors is greater than the number of compensators,
wherein the or at least one compensator (11) is part of at least two different detector-compensator pairs (P.1, ..., P.8),
**characterized in that**
the gas detection device (100) is configured to switch on and switch off each detector-compensator pair (P.1, ..., P.8) independently of any other detector-compensator pair,
wherein the gas detection device (100) is configured such that, at any point in time, at most one detector-compensator pair (P.1, ..., P.8) is switched on and the or any other detector-compensator pair is switched off,
wherein, when the detector-compensator pair (P.1, ..., P.8) is switched on, an electrical voltage (U10) is applied continuously or in a pulsed manner to the detector (10.m, 10.p, 10.1, ..., 10.8), at least temporarily, and an electrical voltage (U11) is applied continuously or in a pulsed manner to the compensator (11.m, 11.p, 11), at least temporarily,
wherein the electrical voltages (U10, U11) applied in each case cause the detector (10.m, 10.p, 10.1, ..., 10.8) to heat up and cause the compensator (11.m, 11.p, 11) to heat up,
wherein each detector (10.m, 10.p, 10.1, ..., 10.8) is configured such that heating the detector (10.m, 10.p, 10.1, ..., 10.8) causes oxidation of the target gas (CH₄), wherein the oxidation releases thermal energy which acts on the detector (10.m, 10.p, 10.1, ..., 10.8),
wherein the or each compensator (11.m, 11.p, 11) is configured such that heating the or each compensator (11.m, 11.p, 11) causes less oxidation of the target gas (CH₄) than heating a detector (10.m, 10.p, 10.1, ..., 10.8), or even causes no oxidation at all,
wherein each detector-compensator pair (P.1, ..., P.8) has a detection variable (ΔU, U10-U11) which correlates with the thermal energy released by the detector-compensator pair (P.1, ..., P.8) during oxidation,
wherein the measuring unit (40, 40.10, 40.11, 41, 41.10, 41.11) is configured to measure, for each detector-compensator pair (P.1, ..., P.8), what value the detection variable (ΔU, U10-U11) for this detector-compensator pair (P.1, ..., P.8) assumes,
wherein the evaluation unit (6) is configured
- to automatically decide if the target gas (CH₄) is present and/or to determine the target gas concentration for each switched-on detector-compensator pair (P.1, ..., P.8), depending on the measured value which the detection variable (ΔU, U10-U11) for the detector-compensator pair (P.1, ..., P.8) assumes, and
wherein the gas detection device (100) is configured
- to switch on each detector-compensator pair (P.1, ..., P.8),
- to effect, for the switched-on detector-compensator pair (P.1, ..., P.8), a measurement of the relevant detection variable (ΔU, U10-U11) and an evaluation, and
- to switch the detector-compensator pair (P.1, ..., P.8) off again.

2. Gas detection device (100) according to any of the preceding claims, **characterized in that**
during a period of use of the gas detection device (100), at least one first detector-compensator pair (P.1, ..., P.7) is switched on for a longer period of time overall than a second detector-compensator pair (P.8).

3. Gas detection device (100) according to claim 2,
**characterized in that**
the evaluation unit (6) is configured
to determine the target gas concentration,
to compare the estimated value of the target gas concentration provided by the first detector-compensator pair (P.1, ..., P.7) with the estimated value of the target gas concentration provided by the second detector-compensator pair (P.8), and
then, if the two estimated values differ from one another by more than a predefined limit, to trigger one of the following steps,
- changing the value of a parameter, wherein the evaluation unit (6) uses this parameter value in order to decide if the target gas (CH₄) is present and/or to determine the target gas concentration, depending on the measured values of the first detector-compensator pair (P.1, ..., P.7), or
- deactivating the first detector-compensator pair (P.1, ..., P.7).

4. Gas detection device (100) according to claim 2 or claim 3,
**characterized in that**
the gas detection device (100) is configured,
during the period of use, to switch the first detector-compensator pair (P.1, ..., P.7) on and off again N1 times and to switch the second detector-compensator pair (P.8) on and off again N2 times,
wherein N1 and N2 are two numbers and N1 is greater than N2.

5. Gas detection device (100) according to any of claims 2 to 4,
**characterized in that**
the gas detection device (100) is configured to carry out a sequence at least once during the period of use,
wherein, during the sequence,
- first, the first detector-compensator pair (P.1, ..., P.7) is switched on and off again N times, and
- then, the second detector-compensator pair (P.8) is switched on and off again M times,
wherein M and N are two numbers and 0 < M < N.

6. Gas detection device (100) according to any of the preceding claims,
**characterized in that**
at least one detector-compensator pair (P.1, ..., P.8) is configured such that the two detection units (10, 11) of the detector-compensator pair (P.1, ..., P.8) each have a detection variable (U10, U11),
wherein the relevant detection variable (U10, U11) correlates with a temperature of the detection unit (10, 11), and
wherein the measuring unit is configured, when the detector-compensator pair (P.1, ..., P.8) is switched on, to measure what value the detection variable for the detector (10.1, ..., 10.8) (U10) assumes, and what value the detection variable (U11) for the compensator (11) assumes.

7. Gas detection device (100) according to claim 6,
**characterized in that**
the gas detection device (100) is configured to operate the detector-compensator pair (P.1, ..., P.8) with the two detection variables (U10, U11)
such that first the one detection unit (10.1, ..., 10.8) and then the other detection unit (11) of the detector-compensator pair (P.1, ..., P.8) is switched on and off again,
such that, at any point in time in an activation period in which the detector-compensator pair (P.1, ..., P.8) is switched on,
at most one detection unit (10.1,..., 10.8, 11) of the detector-compensator pair (P.1, ..., P.8) is switched on.

8. Gas detection device (100) according to claim 6 or claim 7,
**characterized in that**
the gas detection device (100) is configured to operate the detector-compensator pair (P.1, ..., P.8) with the two detection variables (U10, U11)
such that the measuring unit (40, 40.10, 40.11, 41, 41.10, 41.11), in an activation period in which the detector-compensator pair (P.1, ..., P.8) is switched on,
measures the value of the detection variable (U10) of the detector (10.1, ..., 10.8) more often than the value of the detection variable (U11) of the compensator (11),
preferably measures the value of the detection variable (U11) of the compensator (11) exactly once.

9. Gas detection device (100) according to any of the preceding claims,
**characterized in that**
the detection arrangement (DA) extends in a plane, and the maximum dimension perpendicular to this plane is at most 20%, preferably at most 10%, particularly preferably at most 5% of the maximum dimension in the plane,
wherein, viewed in a viewing direction perpendicular to the plane, the detection units (10.m, 10.p, 10.1, ..., 10.8, 11.m, 11.p, 11) are arranged in at least one row, without overlapping.

10. Gas detection device (100) according to claim 9,
**characterized in that**
in the or a row in which a plurality of detection units (10.m, 10.p, 10.4, 10.5, 10.2, 10.7, 11.m, 11.p, 11) are arranged, the or a compensator (11) is arranged between two detectors (10.4, 10.5, 10.2, 10.7).

11. Gas detection device (100) according to any of the preceding claims,
**characterized in that**
the detection arrangement (DA) comprises at least three detectors (10.1, ..., 10.8),
wherein the at least three detectors (10.1, ..., 10.8) and the or a compensator (11) extend in the same plane, and
in this plane the or a compensator (11) is arranged between at least two (10.4, 10.5, 10.2, 10.7) of the at least three detectors (10.1, ..., 10.8).

12. Gas detection device (100) according to any of the preceding claims,
**characterized in that**
the detection arrangement comprises a circuit board (31) and
each detection unit (10.m, 11.m) comprises a heating element (32),
wherein the heating element (32) of a detection unit (10.m, 11.m) heats up if an electrical voltage (U10, U11) is applied to the detection unit (10.m, 11.m),
wherein the heating elements (32) are arranged on and/or in the circuit board (31),
wherein the circuit board (31) provides an electrical contact (46) for each heating element (32), and
wherein the relevant heating element (32) of each detector (10.m) is covered with a coating (35) which comprises a catalytic material or to which a catalytic material (24) is applied.

13. Gas detection device (100) according to any of the preceding claims,
**characterized in that**
the detection arrangement (DA) comprises a third detector-compensator pair (P.8) and the evaluation unit (6) is configured to determine the target gas concentration,
wherein the evaluation unit (6) is configured
- to compare the estimated value of the target gas concentration provided by the first detector-compensator pair (P.1) with the estimated value of the target gas concentration provided by the second detector-compensator pair (P.2, ..., P.7), and
- to switch the third detector-compensator pair (P.8) on, at least temporarily, only if the two estimated values differ from one another by more than a predefined limit.

14. Gas detection device (100) according to claim 13,
**characterized in that**
each detector (10.m, 10.p, 10.1, ..., 10.8) has an effective surface which comes into contact with a gas sample,
wherein the detector (10.8) of the third detector-compensator pair (P.8) has a larger effective surface than the detector of the first detector-compensator pair (P.1) and than the detector of the second detector-compensator pair (P.2, ..., P.8).

15. Gas detection device (100) according to any of the preceding claims,
**characterized in that**
a zero value is predefined for each detector-compensator pair (P.1, ..., P.8), which zero value is a value which the detection variable (ΔU, U10-U11) for this detector-compensator pair (P.1, ..., P.8) assumes in the absence of the target gas (CH₄),
wherein the evaluation unit (6) is configured to calculate the difference between the value which the detection variable (ΔU, U10-U11) assumes and the predefined zero value of the detection variable for each detector-compensator pair (P.1, ..., P.8), and
to decide if the target gas (CH₄) is present and/or to determine the target gas concentration for each switched-on detector-compensator pair (P.1, ..., P.8), depending on the difference between the measured detection variable value and the zero value.

16. Gas detection method for detecting the presence of at least one combustible target gas (CH₄) in a gas sample and/or for determining the concentration of the target gas (CH₄) in the gas sample using a gas detection device (100) which comprises
- a detection arrangement (DA) having at least three detection units (10.m, 10.p, 10.1, ..., 10.8, 11.m, 11.p, 11), and
- a measuring unit (40, 40.10, 40.11, 41, 41.10, 41.11),
wherein at least two different detection units are detectors (10.m, 10.p, 10.1, ..., 10.8) and at least one additional detection unit is a compensator (11.m, 11.p, 11),
so that at least two different detector-compensator pairs (P.1, ..., P.8) are formed having exactly one detector (10.m, 10.p, 10.1, ..., 10.8) and exactly one compensator (11.m, 11.p, 11) in each case,
wherein the number of detectors is greater than the number of compensators,
wherein the or at least one compensator (11) is part of at least two different detector-compensator pairs (P.1, ..., P.8),
**characterized in that**
the method comprises the automatically performed steps, that
- at least one, preferably each, detector-compensator pair (P.1, ..., P.8) is switched on,
- a measurement of the detection variable (ΔU, U10-U11) and an evaluation is effected for the switched-on detector-compensator pair (P.1, ..., P.8), and
- the detector-compensator pair (P.1, ..., P.8) is switched off again,
wherein, at any point in time, at most one detector-compensator pair (P.1, ..., P.8) is switched on and the or any other detector-compensator pair is switched off,
wherein, when the detector-compensator pair (P.1, ..., P.8) is switched on, an electrical voltage (U10) is applied to the detector (10.m, 10.p, 10.1, ..., 10.8), at least temporarily, and an electrical voltage (U11) is applied to the compensator (11.m, 11.p, 11), at least temporarily,
wherein the electrical voltages (U10, U11) applied in each case cause the detector (10.m, 10.p, 10.1, ..., 10.8) to heat up and cause the compensator (11.m, 11.p, 11) to heat up,
wherein heating the detector (10.m, 10.p, 10.1, ..., 10.8) causes oxidation of the target gas (CH₄), wherein the oxidation releases thermal energy which acts on the detector (10.m, 10.p, 10.1, ..., 10.8),
wherein heating the compensator (11.m, 11.p, 11) causes less oxidation than heating a detector (10.m, 10.p, 10.1, ..., 10.8), or even causes no oxidation at all,
wherein each detector-compensator pair (P.1, ..., P.8) has a detection variable (ΔU, U10-U11) which correlates with the thermal energy released by the detector-compensator pair (P.1, ..., P.8) during oxidation, and
wherein the method comprises the additionally automatically performed steps, that, for each switched-on detector-compensator pair (P.1, ..., P.8),
- the measuring unit (40, 40.10, 40.11, 41, 41.10, 41.11) measures what value the detection variable (ΔU, U10-U11) for this detector-compensator pair (P.1, ..., P.8) assumes, and
- it is decided if the target gas (CH₄) is present and/or the target gas concentration is determined depending on the measured value which the detection variable (ΔU, U10-U11) for the detector-compensator pair (P.1, ..., P.8) assumes.

## Revendications

1. Dispositif de détection de gaz (100) qui est configuré pour détecter la présence d'au moins un gaz cible (CH₄) combustible dans un échantillon de gaz et/ou pour déterminer la concentration du gaz cible (CH₄) dans l'échantillon de gaz,
dans lequel le dispositif de détection de gaz (100) comprend
- un agencement de détection (DA) comportant au moins trois unités de détection (10.m, 10.p, 10.1, ..., 10.8, 11.m, 11.p, 11),
- une unité de mesure (40, 40.10, 40.11, 41, 41.10, 41.11) et
- une unité d'évaluation (6) traitant des signaux ,
dans lequel au moins deux unités de détection différentes sont des détecteurs (10.m, 10.p, 10.1, ..., 10.8) et au moins une autre unité de détection est un compensateur (11.m, 11.p, 11),
de sorte qu'au moins deux paires détecteur-compensateur (P.1, ..., P.8) différentes comportant respectivement exactement un détecteur (10.m, 10.p, 10.1, ..., 10.8) et exactement un compensateur (11.m, 11.p, 11) sont formées,
dans lequel le nombre de détecteurs est supérieur au nombre de compensateurs,
dans lequel le compensateur ou au moins un compensateur (11) appartient à au moins deux paires détecteur-compensateur (P.1, ..., P.8) différentes,
**caractérisé en ce que**
le dispositif de détection de gaz (100) est configuré pour activer et désactiver chaque paire détecteur-compensateur (P.1, ..., P.8) indépendamment de chaque autre paire détecteur-compensateur,
dans lequel le dispositif de détection de gaz (100) est configuré de sorte qu'à chaque moment, au plus une paire de détecteur-compensateur (P.1, ..., P.8) est activée et la ou chaque autre paire détecteur-compensateur est désactivée,
dans lequel, lorsque la paire détecteur-compensateur (P.1, ..., P.8) est activée, une tension électrique (U10) est appliquée au moins temporairement, de manière permanente ou pulsée, au détecteur (10.m, 10.p, 10.1, ..., 10.8) et une tension électrique (U11) est appliquée au moins temporairement, de manière permanente ou pulsée, au compensateur (11.m, 11.p, 11),
dans lequel les tensions électriques (U10, U11) respectivement appliquées provoquent un échauffement du détecteur (10.m, 10.p, 10.1, ..., 10.8) et un échauffement du compensateur (11.m, 11.p, 11),
dans lequel chaque détecteur (10.m, 10.p, 10.1, ..., 10.8) est configuré de sorte qu'un échauffement du détecteur (10.m, 10.p, 10.1, ..., 10.8) provoque une oxydation du gaz cible (CH₄), dans lequel l'oxydation libère de l'énergie thermique qui agit sur le détecteur (10.m, 10.p, 10.1, ..., 10.8),
dans lequel le compensateur ou chaque compensateur (11.m, 11.p, 11) est configuré de sorte qu'un échauffement du compensateur ou de chaque compensateur (11.m, 11.p, 11) entraîne une oxydation du gaz cible (CH₄) inférieure par rapport à l'échauffement d'un détecteur (10.m, 10.p, 10.1, ..., 10.8), voire ne provoque aucune oxydation,
dans lequel chaque paire détecteur-compensateur (P.1, ..., P.8) présente une grandeur de détection (ΔU, U10-U11) qui est en corrélation avec l'énergie thermique libérée lors de l'oxydation par la paire détecteur-compensateur (P.1, ..., P.8),
dans lequel l'unité de mesure (40, 40.10, 40.11, 41, 41.10, 41.11) est configurée pour mesurer, pour chaque paire détecteur-compensateur (P.1, ..., P.8), la valeur que la grandeur de détection (ΔU, U10-U11) adopte pour ladite paire détecteur-compensateur (P.1, ..., P.8),
dans lequel l'unité d'évaluation (6) est configurée pour
- pour chaque paire détecteur-compensateur (P.1, ..., P.8) activée, en fonction de la valeur mesurée que la grandeur de détection (ΔU, U10-U11) adopte pour la paire détecteur-compensateur (P.1, ..., P.8),
décider automatiquement que le gaz cible (CH₄) est présent et/ou déterminer la concentration du gaz cible, et
dans lequel le dispositif de détection de gaz (100) est configuré pour
- activer chaque paire détecteur-compensateur (P.1, ..., P.8),
- pour la paire détecteur-compensateur (P.1, ..., P.8) activée, provoquer une mesure de la grandeur de détection (ΔU, U10-U11) respective et une évaluation et
- désactiver de nouveau la paire détecteur-compensateur (P.1, ..., P.8).

2. Dispositif de détection de gaz (100) selon l'une des revendications précédentes, **caractérisé en ce que**
au cours d'une période d'utilisation du dispositif de détection de gaz (100), au moins une première paire détecteur-compensateur (P.1, ..., P.7) est activée pendant une durée totale plus longue que celle d'une deuxième paire détecteur-compensateur (P.8).

3. Dispositif de détection de gaz (100) selon la revendication 2,
**caractérisé en ce que**
l'unité d'évaluation (6) est configurée pour déterminer la concentration de gaz cible, pour comparer la valeur estimée de concentration de gaz cible fournie par la première paire détecteur-compensateur (P.1, ...p.7) avec la valeur estimée de concentration de gaz cible fournie par la deuxième paire détecteur-compensateur (P.8), et
pour déclencher l'une des étapes si les deux valeurs estimées diffèrent l'une de l'autre de plus d'une limite prédéfinie,
- pour modifier la valeur d'un paramètre, dans lequel l'unité d'évaluation (6) utilise ladite valeur de paramètre afin de décider que le gaz cible (CH₄) est présent et/ou de déterminer la concentration de gaz cible en fonction des valeurs mesurées de la première paire détecteur-compensateur (P.1, ..., P.7), ou
- pour désactiver la première paire détecteur-compensateur (P.1, ..., P.7).

4. Dispositif de détection de gaz (100) selon la revendication 2 ou la revendication 3,
**caractérisé en ce que**
le dispositif de détection de gaz (100) est configuré pour, pendant la période d'utilisation, activer et de nouveau désactiver la première paire détecteur-compensateur (P.1, ..., P.7) N1 fois et la deuxième paire détecteur-compensateur (P.8) N2 fois,
dans lequel N1 et N2 sont deux nombres et N1 est supérieur à N2.

5. Dispositif de détection de gaz (100) selon l'une des revendications 2 à 4,
**caractérisé en ce que**
le dispositif de détection de gaz (100) est configuré pour exécuter au moins une fois une séquence pendant la période d'utilisation,
dans lequel, pendant la séquence,
- d'abord la première paire détecteur-compensateur (P.1, ..., P.7) est activée et désactivée de nouveau N fois
- puis la deuxième paire détecteur-compensateur (P.8) est activée et désactivée de nouveau M fois,
dans lequel M et N sont deux nombres et 0 < M < N.

6. Dispositif de détection de gaz (100) selon l'une des revendications précédentes,
**caractérisé en ce que**
au moins une paire détecteur-compensateur (P.1, ..., P.8) est configurée de sorte que les deux unités de détection (10, 11) de la paire détecteur-compensateur (P.1, ..., P.8) présentent respectivement une grandeur de détection (U10, U11),
dans lequel la grandeur de détection (U10, U11) respective est corrélée avec une température de l'unité de détection (10, 11) et
dans lequel l'unité de mesure est configurée pour mesurer, lorsque la paire détecteur-compensateur (P.1, ..., P.8) est activée, la valeur que la grandeur de détection (U10) adopte pour le détecteur (10.1, ..., 10.8) et la valeur que la grandeur de détection (U11) adopte pour le compensateur (11).

7. Dispositif de détection de gaz (100) selon la revendication 6,
**caractérisé en ce que**
le dispositif de détection de gaz (100) est configuré pour faire fonctionner la paire détecteur-compensateur (P.1, ..., P.8) avec les deux grandeurs de détection (U10, U11) de telle sorte que d'abord, l'une des unités de détection (10.1, ..., 10.8) et ensuite l'autre unité de détection (11) de la paire détecteur-compensateur (P.1, ..., P.8) sont activées et de nouveau désactivées, de sorte qu'à chaque moment au cours d'une période d'activation pendant laquelle la paire détecteur-compensateur (P.1, ..., P.8) est activée, au plus une unité de détection (10.1, ..., 10.8, 11) de la paire détecteur-compensateur (P.1, ..., P.8) est activée.

8. Dispositif de détection de gaz (100) selon la revendication 6 ou la revendication 7,
**caractérisé en ce que**
le dispositif de détection de gaz (100) est configuré pour faire fonctionner la paire détecteur-compensateur (P.1, ..., P.8) avec les deux grandeurs de détection (U10, U11) de telle sorte que l'unité de mesure (40, 40.10, 40.11, 41, 41.10, 41.11) mesure, pendant une période d'activation pendant laquelle la paire détecteur-compensateur (P.1, ..., P.8) est activée,
la valeur de la grandeur de détection (U10) du détecteur (10.1, ..., 10.8) plus souvent que la valeur de la grandeur de détection (U11) du compensateur (11), de préférence mesure exactement une fois la valeur de la grandeur de détection (U11) du compensateur (11).

9. Dispositif de détection de gaz (100) selon l'une des revendications précédentes, **caractérisé en ce que**
l'agencement de détection (DA) s'étend dans un plan et la dimension maximale perpendiculairement audit plan est au maximum de 20 %, de préférence au maximum de 10 %, de manière particulièrement préférée au maximum de 5 %, de la dimension maximale dans le plan,
dans lequel, vues dans une direction d'observation perpendiculaire au plan, les unités de détection (10.m, 10.p, 10.1, ..., 10.8, 11.m, 11.p, 11) sont disposés sans chevauchement en au moins une rangée.

10. Dispositif de détection de gaz (100) selon la revendication 9,
**caractérisé en ce que**
dans la rangée ou dans une rangée dans laquelle plusieurs unités de détection (10.m, 10.p, 10.4, 10.5, 10.2, 10.7, 11.m, 11.p, 11) sont disposées, le compensateur ou un compensateur (11) est disposé entre deux détecteurs (10.4, 10.5, 10.2, 10.7).

11. Dispositif de détection de gaz (100) selon l'une des revendications précédentes, **caractérisé en ce que**
l'agencement de détection (DA) comprend au moins trois détecteurs (10.1, ..., 10.8),
dans lequel les au moins trois détecteurs (10.1, ..., 10.8) et le compensateur ou un compensateur (11) s'étendent dans le même plan et dans ledit plan, le compensateur ou un compensateur (11) est disposé entre au moins deux détecteurs (10.4, 10.5, 10.2, 10.7) parmi les au moins trois détecteurs (10.1, ..., 10.8)

12. Dispositif de détection de gaz (100) selon l'une des revendications précédentes,
**caractérisé en ce que**
l'agencement de détection comprend une carte de circuit imprimé (31) et chaque unité de détection (10.m, 11.m) comprend respectivement un élément chauffant (32),
dans lequel l'élément chauffant (32) d'une unité de détection (10.m, 11.m) s'échauffe lorsqu'une tension électrique (U10, U11) est appliquée à l'unité de détection (10.m, 11.m),
dans lequel les éléments chauffants (32) sont disposés sur et/ou dans la carte de circuit imprimé (31),
dans lequel la carte de circuit imprimé (31) fournit respectivement un contact électrique (46) pour chaque élément chauffant (32) et
dans lequel l'élément chauffant (32) respectif de chaque détecteur (10.m) est recouvert de respectivement un revêtement (35) qui présente un matériau catalytique ou sur lequel est appliqué un matériau catalytique (24).

13. Dispositif de détection de gaz (100) selon l'une des revendications précédentes,
**caractérisé en ce que**
l'agencement de détection (DA) comprend une troisième paire détecteur-compensateur (P.8) et l'unité d'évaluation (6) est configurée pour déterminer la concentration de gaz cible,
dans lequel l'unité d'évaluation (6) est configurée pour
- comparer la valeur estimée de concentration de gaz cible fournie par la première paire détecteur-compensateur (P.1) avec la valeur estimée de concentration de gaz cible fournie par la deuxième paire détecteur-compensateur (P.2, ..., P.7), et
- activer la troisième paire détecteur-compensateur (P.8) au moins temporairement que si les deux valeurs estimées diffèrent l'une de l'autre de plus d'une limite prédéfinie.

14. Dispositif de détection de gaz (100) selon la revendication 13,
**caractérisé en ce que**
chaque détecteur (10.m, 10.p, 10.1, ..., 10.8) présente une surface efficace qui entre en contact avec un échantillon de gaz,
dans lequel le détecteur (10.8) de la troisième paire détecteur-compensateur (P.8) présente une surface efficace plus grande que celle du détecteur de la première paire détecteur-compensateur (P.1) et que celle du détecteur de la deuxième paire détecteur-compensateur (P.2, ..., P.8).

15. Dispositif de détection de gaz (100) selon l'une des revendications précédentes,
**caractérisé en ce que**
pour chaque paire détecteur-compensateur (P.1, ..., P.8), respectivement une valeur zéro est prédéfinie, laquelle est une valeur que la grandeur de détection (ΔU, U10-U11) adopte pour ladite paire détecteur-compensateur (P.1, ..., P.8) en l'absence du gaz cible (CH₄),
dans lequel l'unité d'évaluation (6) est configurée pour calculer, pour chaque paire détecteur-compensateur (P.1, ..., P.8), la différence entre la valeur adoptée par la grandeur de détection (ΔU, U10-U11) et la valeur zéro prédéfinie de la grandeur de détection, et
pour chaque paire détecteur-compensateur (P.1, ..., P.8) activée, en fonction de la différence entre la valeur de la grandeur de détection mesurée et la valeur zéro, décider que le gaz cible (CH₄) est présent et/ou déterminer la concentration de gaz cible.

16. Procédé de détection de gaz permettant de détecter la présence d'au moins un gaz cible (CH₄) combustible dans un échantillon de gaz et/ou permettant de déterminer la concentration du gaz cible (CH₄) dans l'échantillon de gaz en utilisant un dispositif de détection de gaz (100) qui comprend
- un agencement de détection (DA) comportant au moins trois unités de détection (10.m, 10.p, 10.1, ..., 10.8, 11.m, 11.p, 11) et
- une unité de mesure (40, 40.10, 40.11, 41, 41.10, 41.11) ,
dans lequel au moins deux unités de détection différentes sont des détecteurs (10.m, 10.p, 10.1, ..., 10.8) et au moins une autre unité de détection est un compensateur (11.m, 11.p, 11),
de sorte qu'au moins deux paires détecteur-compensateur (P.1, ..., P.8) différentes comportant respectivement exactement un détecteur (10.m, 10.p, 10.1, ..., 10.8) et exactement un compensateur (11.m, 11.p, 11) sont formées,
dans lequel le nombre de détecteurs est supérieur au nombre de compensateurs,
dans lequel le compensateur ou au moins un compensateur (11) appartient à au moins deux paires détecteur-compensateur (P.1, ..., P.8) différentes,
**caractérisé en ce que**
le procédé comprend les étapes exécutées automatiquement selon lesquelles
- au moins une, de préférence chaque paire détecteur-compensateur (P.1, ..., P.8), est activée,
- pour la paire détecteur-compensateur (P.1, ..., P.8) activée, une mesure de la grandeur de détection (ΔU, U10-U11) et une évaluation sont effectuées et
- la paire détecteur-compensateur (P.1, ..., P.8), est de nouveau désactivée,
dans lequel, à chaque moment, au plus une paire détecteur-compensateur (P.1, ..., P.8) est activée et la ou chaque autre paire détecteur-compensateur est désactivée,
dans lequel, lorsque la paire détecteur-compensateur (P.1, ..., P.8) est activée, une tension électrique (U10) est appliquée au moins temporairement au détecteur (10.m, 10.p, 10.1, ..., 10.8) et une tension électrique (U11) est appliquée au moins temporairement au compensateur (11.m, 11.p, 11),
dans lequel les tensions électriques (U10, U11) respectivement appliquées provoquent un échauffement du détecteur (10.m, 10.p, 10.1, ..., 10.8) et un échauffement du compensateur (11.m, 11.p, 11),
dans lequel un échauffement du détecteur (10.m, 10.p, 10.1, ..., 10.8) provoque une oxydation du gaz cible (CH₄), dans lequel l'oxydation libère de l'énergie thermique qui agit sur le détecteur (10.m, 10.p, 10.1, ..., 10.8),
dans lequel un échauffement du compensateur (11.m, 11.p, 11) entraîne une oxydation inférieure par rapport à l'échauffement d'un détecteur (10.m, 10.p, 10.1, ..., 10.8), voire ne provoque aucune oxydation,
dans lequel chaque paire détecteur-compensateur (P.1, ..., P.8) présente une grandeur de détection (ΔU, U10-U11) qui est corrélée avec l'énergie thermique libérée lors de l'oxydation par la paire détecteur-compensateur (P.1, ..., P.8), et
dans lequel le procédé comprend les étapes supplémentaires exécutées automatiquement selon lesquelles, pour chaque paire détecteur-compensateur (P.1, ..., P.8) activée,
- l'unité de mesure (40, 40.10, 40.11, 41, 41.10, 41.11) mesure la valeur que la grandeur de détection (ΔU, U10-U11) adopte pour ladite paire détecteur-compensateur (P.1, ..., P.8), et
- en fonction de la valeur mesurée que la grandeur de détection (ΔU, U10-U11) adopte pour la paire détecteur-compensateur (P.1, ..., P.8) activée,
la présence du gaz cible (CH₄) est décidée et/ou la concentration de gaz cible est déterminée.
